(19) Europäisches Patentamt
European Patent Office
Office européen des brevets

(11) EP 1 609 459 B1

(12) FASCICULE DE BREVET EUROPEEN

(45) Date de publication et mention de la délivrance du brevet:
**08.04.2009 Bulletin 2009/15**

(51) Int Cl.:
***A61K 8/49*** *(2006.01)* ***A61Q 5/06*** *(2006.01)*

(21) Numéro de dépôt: **05291344.9**

(22) Date de dépôt: **23.06.2005**

# (54) Utilisation de composés polycationiques en teinture des fibres kératiniques

Verwendung von polykationischen Verbindungen zur Färbung der Keratinfasern

Use of polycationic compounds for dyeing keratinous fibers

(84) Etats contractants désignés:
**AT BE BG CH CY CZ DE DK EE ES FI FR GB GR HU IE IS IT LI LT LU MC NL PL PT RO SE SI SK TR**

(30) Priorité: **23.06.2004 FR 0406851**

(43) Date de publication de la demande:
**28.12.2005 Bulletin 2005/52**

(73) Titulaire: **L'ORÉAL**
**75008 Paris (FR)**

(72) Inventeur: **Lagrange, Alain**
**77700 Coupvray (FR)**

(74) Mandataire: **Bulle, Françoise**
**Bureau Casalonga & Josse**
**Bayerstrasse 71/73**
**80335 München (DE)**

(56) Documents cités:
**EP-A- 1 020 176**
**EP-A- 1 415 643**
**EP-A- 1 428 505**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 609 459 B1

## Description

La présente demande a pour objet l'utilisation de composés polycationiques particuliers, à titre de colorants directs dans des compositions tinctoriales pour la teinture des fibres kératiniques, et en particulier les cheveux humains. Elle vise également des compositions tinctoriales à base de ces colorants, ainsi que l'utilisation de ces compositions pour la teinture des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho-ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Ces colorants, insolubles dans le milieu de teinture, sont piégés à l'intérieur du cheveu.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser poser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes peuvent être mises en oeuvre sans la présence d'un agent oxydant. Ces teintures peuvent être effectuées de manière répétée sans dégrader la fibre kératinique.

Il est connu par exemple d'utiliser des colorants directs nitrés benzéniques, anthraquinoniques, nitropyridiniques, azoïques, xanthéniques, acridiniques, aziniques ou triarylméthaniques.

Il en résulte des colorations particulièrement chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration. Ces colorants directs sont en outre généralement sensibles à la lumière car la résistance du chromophore vis-à-vis des attaques photochimiques est faible, ce qui conduit à un affadissement de la coloration des cheveux dans le temps. La sensibilité de ces colorants à la lumière dépend de leur répartition uniforme ou en agrégats dans et/ou sur la fibre kératinique.

Par ailleurs, les colorants directs classiques ne sont pas toujours complètement inoffensifs, c'est pourquoi en cosmétique capillaire, on recherche des molécules colorantes de ce type toujours plus performantes en terme d'innocuité.

Des compositions tinctoriales pour les cheveux contenant des colorants directs cationiques sont décrites dans les documents EP 1 020 176 A2, EP 1 428 505 A1 et EP 1 415 643 A1.

Il existe un réel besoin de disposer de compositions de teinture directe améliorées en terme de tenue aux shampooings et de montée du colorant.

De manière surprenante et avantageuse, la Demanderesse vient de découvrir que l'utilisation de composés polycationiques particuliers dans des compositions pour la teinture des fibres kératiniques, en particulier des fibres kératiniques humaines telles que les cheveux, permettait d'obtenir des compositions tinctoriales qui présentent ces améliorations.

Outre leur avantage en terme d'innocuité, les compositions selon la présente demande permettent l'obtention de teintures résistantes aux agents extérieurs (tels que le soleil, les intempéries), ainsi qu'aux shampooings et à la transpiration, et permettent d'obtenir des nuances intenses et tenaces sur les fibres.

La présente invention a ainsi pour objet l'utilisation à titre de colorant direct dans des compositions tinctoriales pour fibres kératiniques, en particulier les fibres kératiniques humaines telles que les cheveux, ou pour la fabrication de telles compositions, de composés de formule (I) décrite ci-dessous.

L'invention a également pour objet une composition tinctoriale pour la teinture des fibres kératiniques, en

particulier des fibres kératiniques humaines telles que les cheveux comprenant dans un milieu de teinture approprié au moins une base d'oxydation et au moins un composé de formule (I).

Un autre objet de l'invention porte sur un procédé de teinture des fibres kératiniques, en particulier des fibres kératiniques humaines telles que les cheveux mettant en oeuvre cette composition.

L'invention a également pour objet l'utilisation de la composition de la présente invention sur les fibres kératiniques, en particulier les fibres kératiniques humaines telles que les cheveux pour obtenir des teintures présentant une bonne résistance aux agents extérieurs et aux shampooings. Les formes tautomères des composés de formule (I) sont également utilisables.

D'autres caractéristiques, aspects, objets et avantages de l'invention apparaîtront encore plus clairement à la lecture de la description et des exemples qui suivent.

Les composés utilisables à titre de colorants directs selon l'invention répondent à la formule (I),

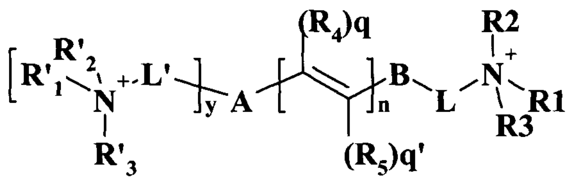

$zX^{z'-}$

(I)

dans laquelle :

y désigne 0 ou 1 ;

n désigne un nombre entier allant de 1 à 4 ;

q et q' désignent indépendamment l'un de l'autre 0 ou 1 ;

$X^{z'-}$ désigne un anion organique ou minéral ;

z et z' sont tels que la charge globale de la molécule soit nulle ;

un seul ou les deux atomes de carbone extrêmes du groupement

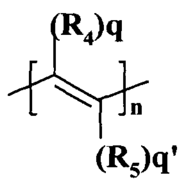

font partie des cycles A et/ou B ;

A et/ou B désignent indépendamment l'un de l'autre un groupement cyclique non hétérocyclique aromatique ou un groupement hétérocyclique comportant de 5 à 15 chaînons, substitué ou non, l'un au moins des groupements A ou B étant porteur d'au moins une charge positive à la condition que si A est un cycle non-hétérocyclique, alors B désigne un groupement benzothiazolium, naphthothiazolium, naphthoxazolium, benzoxazolium, benzimidazolium, thiazolium, pyrimidobenzimidazolium, benzopyrroloimidazolium, pyrido benzoxazolium, pyrido benzothiazolium, pyrrolo benzoxazolium, pyrrolo benzothiazolium, naphtothiazolo pyridinium, azépino thiazolium, diazepino benzoxazolium, azepino benzoxazolium, pyrido benzoxazolium, azocino benzoxazolium, azocino benzothiazolium, azepino benzothiazolium, ou quinolinium. De préférence A et B représentent des hétérocycles.

L et L' désignent indépendamment l'un de l'autre une chaîne alkylène linéaire, ramifiée ou cyclique comportant

de 1 à 30 atomes de carbone et/ou une chaîne aromatique comportant de 6 à 30 atomes de carbone, substituée ou non ; la chaîne pouvant être ou non interrompue ou terminée par un ou plusieurs groupements hétéroatomiques choisi parmi O, S, NH, NR avec R représentant un radical alkyle en C1-C20, linéaire ou ramifié éventuellement substitué, ou interrompue par un ou plusieurs radicaux cycliques, aromatiques ou non, hétérocycliques ou non, la chaîne elle-même pouvant être porteuse d'une ou plusieurs charges cationiques quaternaires et un ou plusieurs atomes de carbone de la chaîne pouvant être remplacés par un groupement carbonyle.

R1, R2, R3, R'1, R'2, et R'3 désignent indépendamment l'un de l'autre un radical alkyle en C1-C20 linaire, ramifié ou cyclique éventuellement substitué, deux de ces radicaux ou plus pouvant former avec l'atome d'azote les portant et éventuellement l'un des atomes de carbone ou d'azote de L pour R1, R2 et/ou R3 ou L' pour R'1, R'2 et/ou R'3, un hétérocycle saturé, insaturé ou aromatique, éventuellement substitué pouvant contenir un ou plusieurs hétéroatomes supplémentaires ; un radical aryle en C6-C30 éventuellement substitué.

Le radical porté par un atome d'azote quaternisé ou non engagé dans un hétérocycle saturé ou non, aromatique ou non, représente un radical alkyle en C1-C20 linaire, ramifié ou cyclique, éventuellement substitué.

R, R4 et R5 désignent indépendamment l'un de l'autre un atome d'hydrogène, un atome d'halogène, un radical aryle en C6-C30 éventuellement substitué, un radical alkyle en C1-C10 linaire, ramifié ou cyclique éventuellement substitué ; les radicaux R4 et R5 peuvent former avec les atomes de carbone les portant un cycle de 5 à 10 chaînons.

Par substituant, on entend au sens de la présente invention un radical choisi parmi les radicaux hydroxy, amino, nitro, halogène, alkyle en C1-C10, aryle en C6-C30, hydroxyalkyle en C1-C10, mono alkyle(C1-C10)amino ou di alkyle (C1-C10)amino pouvant éventuellement former avec l'atome d'azote auquel chacun est lié, un hétérocycle saturé ou non, comprenant éventuellement au moins un autre hétéroatome, de préférence l'azote, mono ou di hydroxyalkyle(C1-C10)amino, trialkyl(C1-C10)ammonium, alcoxy en C1C10 éventuellement substitué par un groupement trialkyl(C1-C10) ammonium, amido, phénylamido, carboxy sous forme d'acide ou de sel, alcoxy(C1-C10)carbonyle, sulfonato, alcoxy (C1-C10)carbonylalkyle(C1-C10), amidoalkyl(C1-C10)amido, difluorométhane sulfonyle, hétérocycle de 5 à 10 chaînons, hétérocycle alkyle en C1-C10 avec un hétérocycle ayant de 5 à 10 chaînons.

A titre d'hétérocycles possibles pour A et B autres que ceux mentionnés ci-dessus on peut citer les cycles imidazolium, pyridinium, triazolium, pyrazolium, pyrimidinium, indolium, indolinium, benzoselenazolium.

A titre d'hétérocycles non cationiques, on peut citer les équivalents non cationiques des hétérocycles listés précédemment, mais aussi le (3H) benzothiazolyle, le (3H)benzoxazolyle, le (2H) indolyle, le (1H) quinolinyle, le (1H) pyridinyle, le thiazolidinyle, le (4H)pyriidnyle, le (4H)pyrimidinyle.

A titre de cycles non hétérocycliques aromatiques on peut citer les cycles benzénique, naphtalénique, anthracéniques, ces cycles pouvant être éventuellement substitués par un ou plusieurs des substituants mentionnés ci-dessus.

A titre d'anions organiques ou minéraux on peut citer les ions halogénures et en particulier les ions chlorure, bromure, iodure, les ions méthanesulfonate, les ions méthosulfate, les ions bromate ou chlorate, les ions acétate, les ions sulfate, les ions tartrate, les ions lactate, les ions citrates.

Parmi les composés de formule (I), on peut citer les composés suivants. La nature du contre-ion $X^{z'-}$ n'est pas critique.

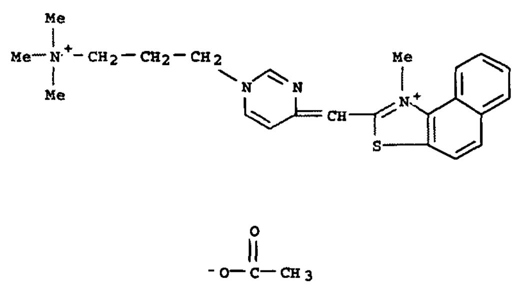

Acétate de Triméthyl-{3-[4-(1-méthyl-naphtho[1,2-d]thiazolium-2-ylméthylène)-4H-pyrimidin-1-yl]-propyl}-ammonium

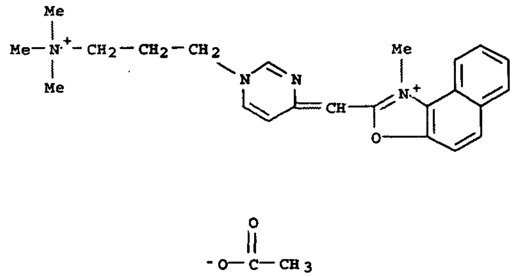

Acétate de Triméthyl-{3-[4-(1-méthyl-naphtho[1,2-d]oxazolium-2-ylméthylène)-4H-pyrimidin-1-yl]-propyl}-ammonium

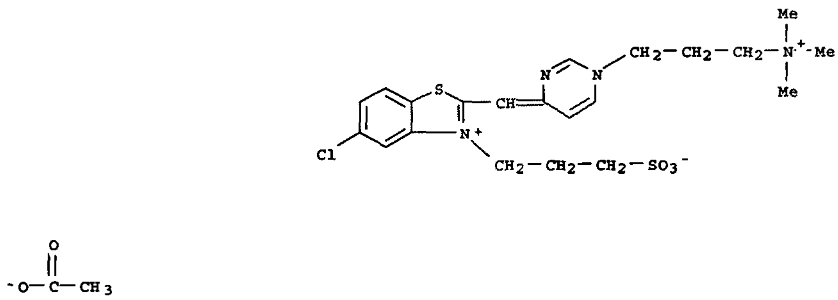

acétate de (3- {4-[5-chloro-3-(3-sulfo-propyl)-benzothiazolium-2-ylméthylène]- 4H-pyrimidin-1-yl]-propyl)-ammonium

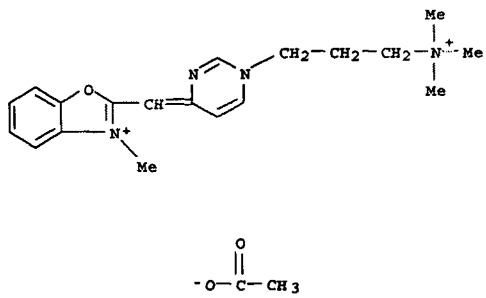

Acétate de triméthyl-{3-[4-(3-méthyl-benzooxazolium-2-ylméthylène)-4H-pyrimidin-1-yl]-propyl}-ammonium

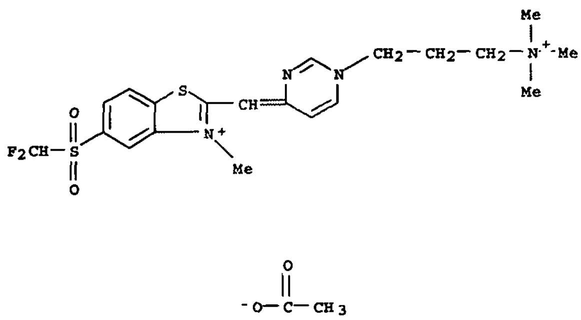

Acétate de (3-{4-[5-(difluoro-méthanesulfonyl)-3-méthyl-benzothiazol-2-ylméthylène]-4H-pyrimidin-1-yl]-propyl}-ammonium

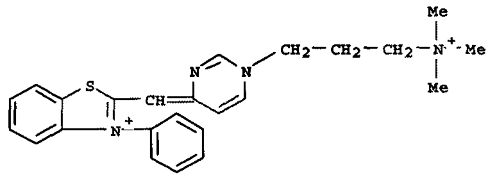

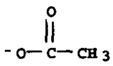

Acétate de triméthyl-{3-[4-(3-phényl-benzothiazol-2-ylméthylène)-4H-pyrimidin-1-yl]-propyl}-ammonium

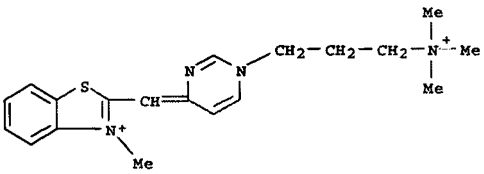

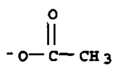

Acétate de triméthyl-{3-[4-(3-méthyl-benzothiazolium-2-ylméthylène)-4H-pyrimidin-1-yl]-propyl}-ammonium

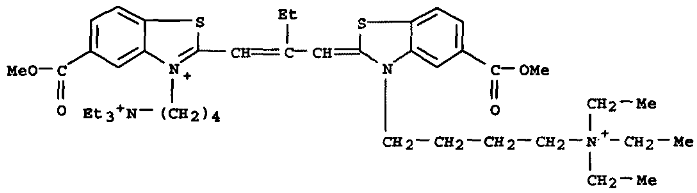

· 3 $Br^-$

tribromure de 5-(méthoxycarbonyl)-2-[2-[[5-(méthoxycarbonyl)-3-[4-(triéthylammonio)butyl]-2(3H)-benzothiazolylidene] méthyl]-1-butenyl]-3-[4triéthylammonio)butyl]- benzothiazolium

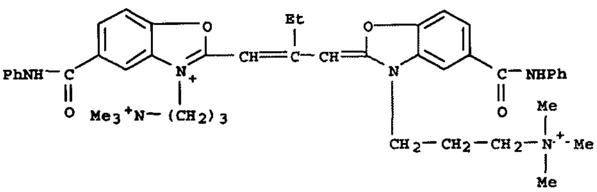

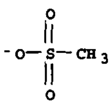

triméthanesulfonate de 5-[(phénylamino)carbonyl]-2-[2-[[5-[(phénylamino)carbonyl]-3-[3-(triméthylammonio)propyl]-2(3H)-benzoxazolylidene]méthyl]-1-butenyl]-3-[3-(triméthylammonio)propyl]-benzoxazolium

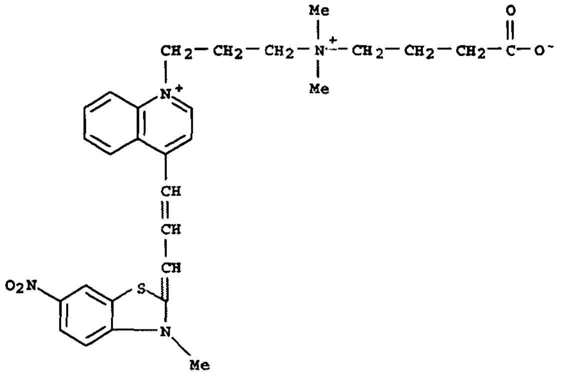

Quinolinium, 1-[3-[(3-carboxypropyl)diméthylammonio]propyl]-4-[3-(3-méthyl-6-nitro-2(3H)-benzothiazolylidene)-1-propényl]-, mono(inner salt) (9CI)

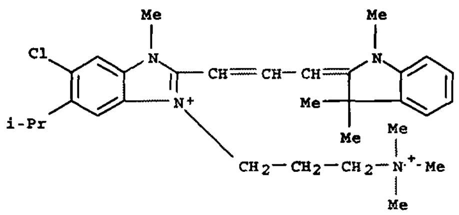

1H-Benzimidazolium,5-chloro-2-[3-(1,3-dihydro-1,3,3-triméthyl-2H-indol-2-ylidène)-1-propényl]-3-méthyl-6-(1-méthyléthyl)-1-[3-(triméthylammonio)propyl]- (9CI)

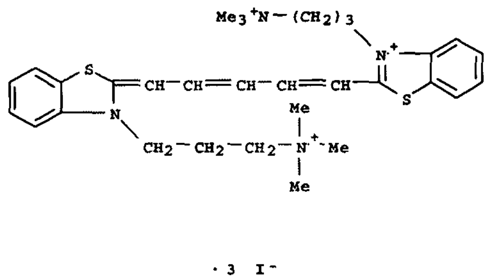

triiodure de 3-[3-(trimethylammonio)propyl]-2-[5-[3-[3-(trimethylammonio)propyl]-2(3H)-benzothiazolylidene]-1,3-pentadienyl]-benzothiazolium

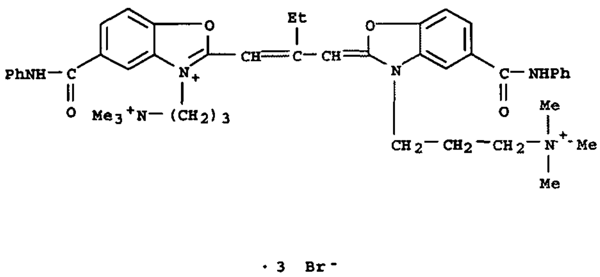

tribromure de 5-[(phénylamino)carbonyl]-2-[2-[[5-[(phénylamino)carbonyl]-3-[3-(triméthylammonio)propyl]-2(3H)-benzoxazolylidene]méthyl]-1-butényl]-3-[3-(triméthylammonio)propyl]-benzoxazolium

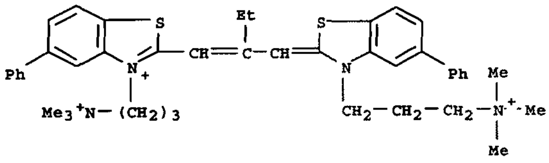

$\cdot 3\ Br^-$

tribromure de 5-phényl-2-[2-[[5-phényl-3-[3-(triméthylammonio)propyl]-2(3H)-benzothiazolylidene]méthyl]-1-butényl]-3-[3-(triméthylammonio)propyl]-benzothiazolium

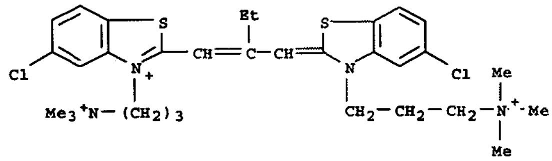

$\cdot 3\ Br^-$

tribromure de 5-chloro-2-[2-[[5-chloro-3-[3-(triméthylammonio)propyl]-2(3H)-benzothiazolylidene]méthyl]-1-butényl]-3-[3-(triméthylammonio)propyl]-benzothiazolium

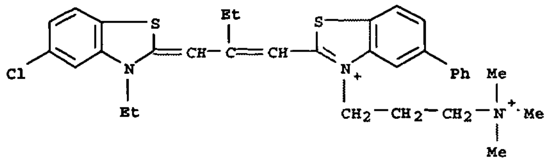

$\cdot 2\ Br^-$

dibromure de 2-[2-[(5-chloro-3-éthyl-2(3H)-benzothiazolylidene)méthyl]-1-butényl]-5-phényl-3-[3-(triméthylammonio)propyl]-benzothiazolium

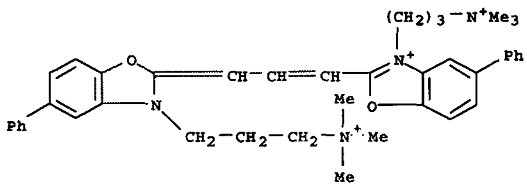

$\cdot 3\ Br^-$

tribromure de 5-phényl-2-[3-[5-phényl-3-[3-(triméthylammonio)propyl]-2(3H)-benzoxazolylidene]-1-propényl]-3-[3-(triméthylammonio)propyl]-benzoxazolium

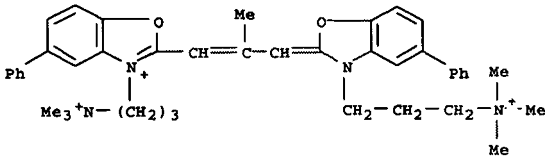

$\cdot 3\ Br^-$

tribromure de 2-[2-méthyl-3-[5-phényl-3-[3-(triméthylammonio)propyl]-2(3H)-benzoxazolylidene]-1-propényl]-5-phényl-3-[3-(triméthylammonio)propyl]-benzoxazolium

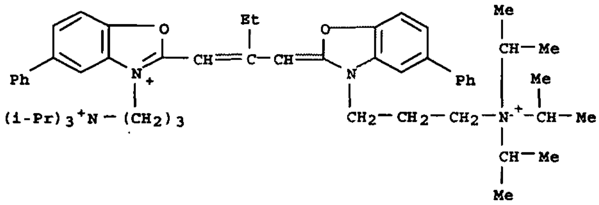

$\cdot 3\ Br^-$

tribromure de 5-phényl-2-[2-[[5-phényl-3-[3-[tris(1-méthyléthyl)ammonio]propyl]-2(3H)-benzoxazolylidene]méthyl]-1-butényl]-3-[3-[tris(1-méthyléthyl)ammonio]propyl]-benzoxazolium

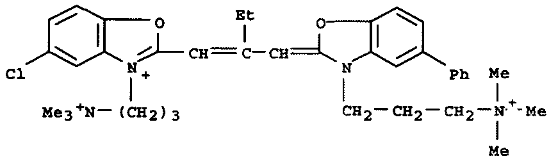

$\cdot 3\ Br^-$

tribromure de 5-chloro-2-[2-[[5-phényl-3-[3-(triméthylammonio)propyl]-2(3H)-benzoxazolylidene]méthyl]-1-buténoyl]-3-[3-(triméthylammonio)propyl]-benzoxazolium

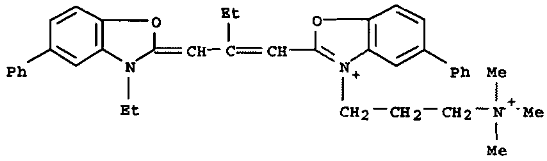

$\cdot 2\ Br^-$

dibromure de 2-[2-[(3-éthyl-5-phényl-2(3H)-benzoxazolylidene)méthyl]-1-butényl]-5-phényl-3-[3-(triméthylammonio)propyl]-benzoxazolium

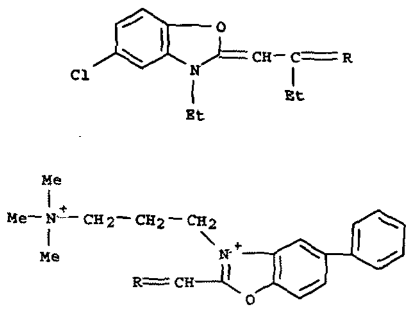

· 2 $Br^-$

dibromure de 2-[2-[(5-chloro-3-éthyl-2(3H)-benzoxazolylidene)méthyl]-1-butényl]-5-phényl-3-[3-(triméthylammonio) propyl]-benzoxazolium,

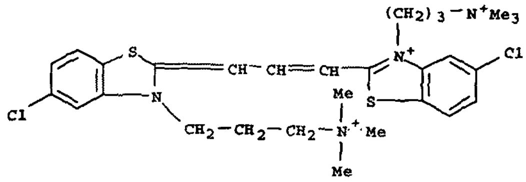

· 3 $Br^-$

tribromure de 5-chloro-2-[3-[5-chloro-3-[3-(triméthylammonio)propyl]-2(3H)-benzothiazolylidene]-1-propényl]-3-[3-(triméthylammonio)propyl]-benzothiazolium

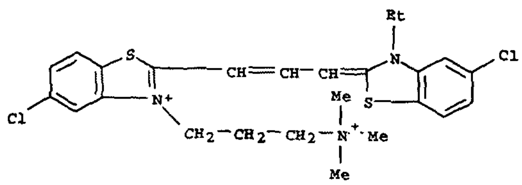

· 2 $Br^-$

dibromure de 5-chloro-2-[3-(5-chloro-3-éthyl-2(3H)-benzothiazolylidene)-1-propényl]-3-[3-(triméthylammonio)propyl]-benzothiazolium

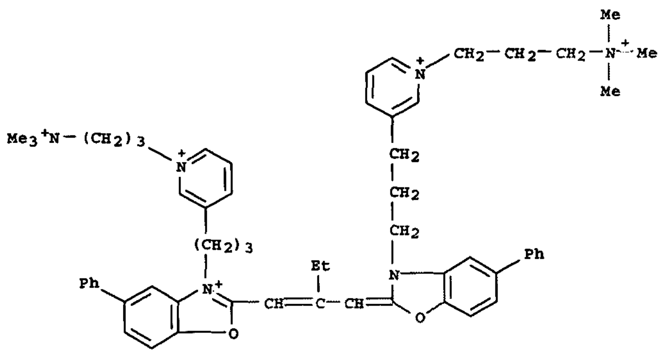

$\cdot 5\ Br^-$

pentabromure de 5-phényl-2-[2-[[5-phényl-3-[3-[1-[3-(triméthylammonio)propyl]pyridinium-3-yl]propyl]-2(3H)-benzoxazolylidene]méthyl]-1-butényl]-3-[3-[1-[3-(triméthylammonio)propyl]pyridinium-3-yl]propyl]-benzoxazolium

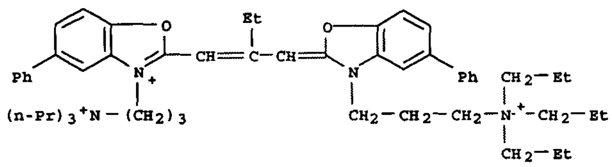

$\cdot 3\ Br^-$

tribromure de 5-phényl-2-[2-[[5-phényl-3-[3-(trpropylammonio)propyl]-2(3H)-benzoxazolylidene]méthyl]-1-butényl]-3-[3-(tripropylammonio)propyl]-benzoxazolium

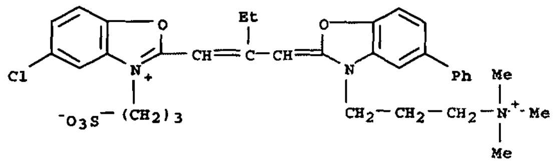

$\cdot\ Br^-$

bromure de 5-chloro-2-[2-[[5-phényl-3-[3-(triméthylammonio)propyl]-2(3H)-benzoxazolylidene]méthyl]-1-butényl]-3-(3-sulfopropyl)-benzoxazolium

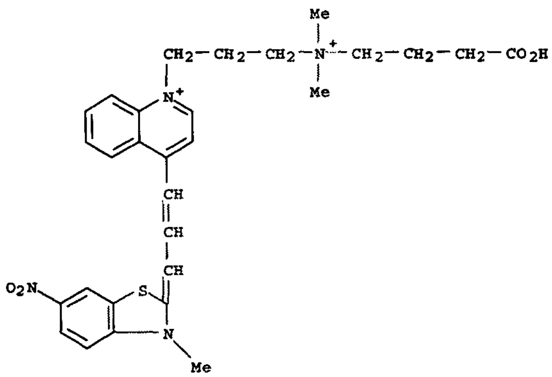

1-[3-[(3-carboxypropyl)diméthylammonio]propyl]-4-[3-(3-méthyl-6-nitro-2(3H)-benzothiazolylidene)-1-propényl]-quinolinium

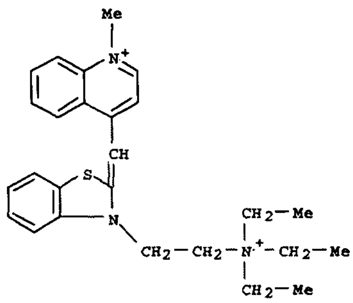

· 2 I⁻

diiodure de 1-méthyl-4-[[3-[2-(triéthylammonio)éthyl]-2(3H)-benzothiazolylidene]méthyl]-quinolinium

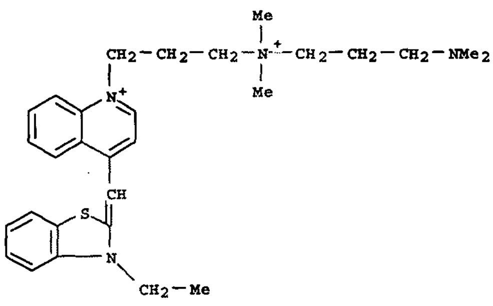

1-[3-[[3-(diméthylamino)propyl]diméthylammonio]propyl]-4-[(3-éthyl-2(3H)-benzothiazolylidene)méthyl]-quinolinium

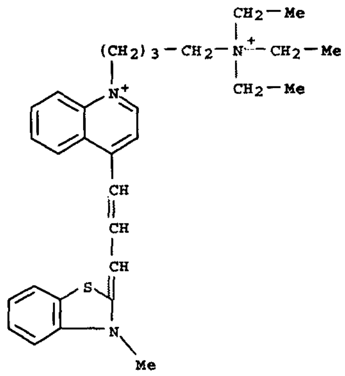

$\cdot 2\ I^-$

diodure de 4-[3-(3-methyl-2(3H)-benzothiazolylidene)-1-propényl]-1-[4-(triéthylammonio)butyl]-quinolinium

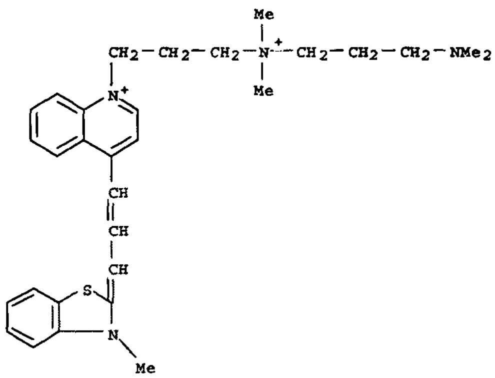

$\cdot 2\ I^-$

diiodure de 1-[3-[[3-(diméthylamino)propyl]diméthylammonio]propyl]-4-[3-(3-méthyl-2(3H)-benzothiazolylidene)-1-propényl]-quinolinium

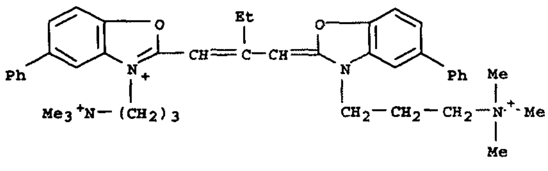

$\cdot 3\ Br^-$

tribromure de 5-phényl-2-[2-[[5-phényl-3-[3-(triméthylammonio)propyl]-2(3H)-benzoxazolylidene]méthyl]-1-buténył]-3-[3-(triméthylammonio)propyl]-benzoxazolium

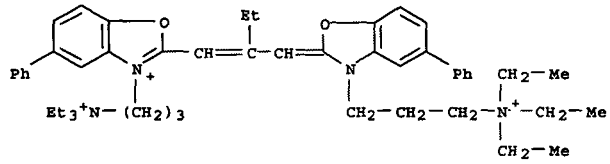

· 3 Br⁻

tribromure de 5-phényl-2-[2-[[5-phényl-3-[3-(triéthylammonio)propyl]-2(3H)-benzoxazolylidene]méthyl]-1-buténуl]-3-[3-(triéthylammonio)propyl]-benzoxazolium

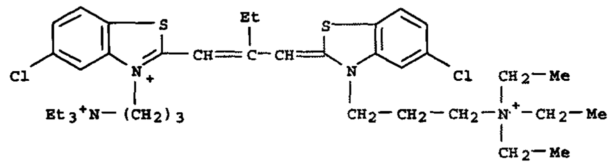

· 3 Br⁻

tribromure de 5-chloro-2-[2-[[5-chloro-3-[3-(triéthylammonio)propyl]-2(3H)-benzothiazolylidene]méthyl]-1-buténуl]-3-[3-(triéthylammonio)propyl]-benzothiazolium

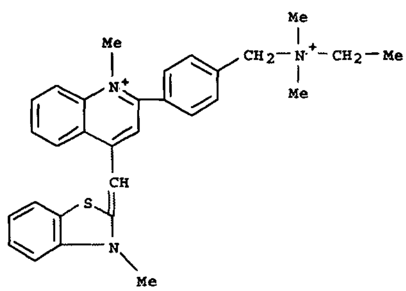

· 2 I⁻

diiodure de 2-[4-[(éthyldiméthylammonio)méthyl]phényl]-1-méthyl-4-[(3-méthyl-2(3H)-benzothiazolylidene)méthyl]-quinolinium

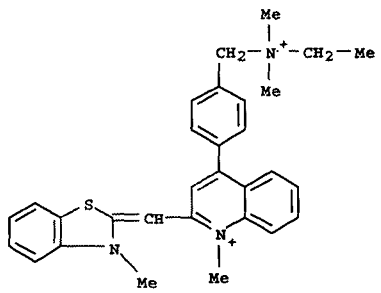

$\cdot 2\ I^-$

diiodure de 4-[4-[(éthyldiméthylammonio)méthyl]phényl]-1-méthyl-2-[(3-méthyl-2(3H)-benzothiazolylidene)méthyl]-quinolinium

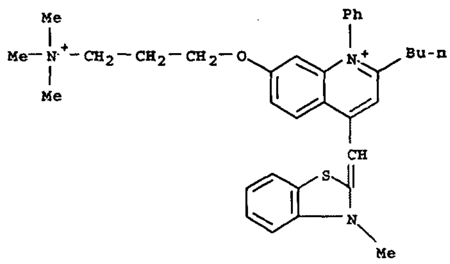

$\cdot 2\ I^-$

diiodure de 2-butyl-4-[(3-méthyl-2(3H)-benzothiazolylidene)méthyl]-1-phényl-7-[3-(triméthylammonio)propoxy]-quinolinium

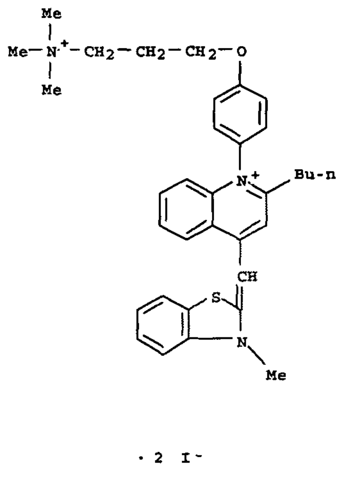

$\cdot 2\ I^-$

diiodure de 2-butyl-4-[(3-méthyl-2(3H)-benzothiazolylidene)méthyl]-1-[4-[3-(triméthylammonio)propoxy]phényl]-quinolinium

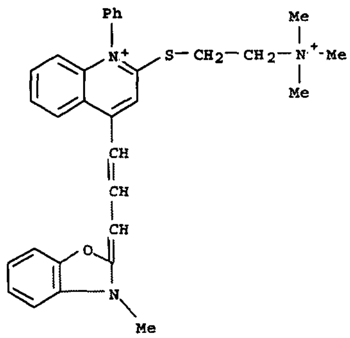

$\cdot 2\ I^-$

diiodure de 4-[3-(3-méthyl-2(3H)-benzoxazolylidene)-1-propényl]-1-phényl-2-[[2-(triméthylammonio)éthyl]thio]-quinolinium

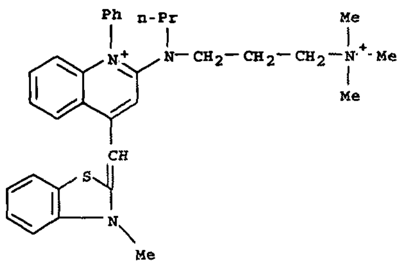

$\cdot 2\ I^-$

diiodure de 4-[(3-méthyl-2(3H)-benzothiazolylidene)méthyl]-1-phéenyl-2-[propyl[3-(triméthylammonio)propyl]amino]-quinolinium

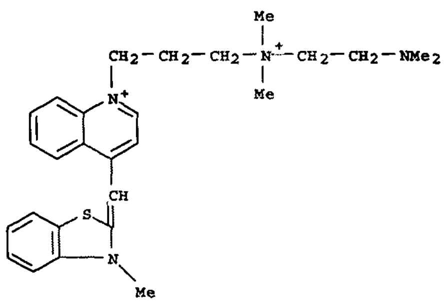

$\cdot 2\ I^-$

$\cdot$ HI

diiodure de 1-[3-[[2-(dimethylamino)ethyl]dimethylammonio]propyl]-4-[(3-methyl-2(3H)-benzothiazolylidene) methyl]-quinolinium, monohydriodure

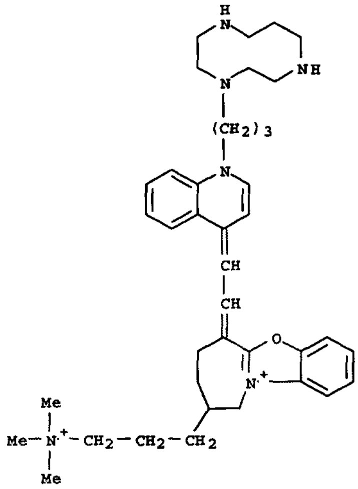

7,8,9,10-tétrahydro-6-[[1-[3-(octahydro-1,4,7-triazecin-4(1H)-yl)propyl]-4(1H)-quinolinylidene]éthylidene]-9-[3-(triméthylammonio)propyl]-6H-azepino[2, 1-b]benzoxazolium

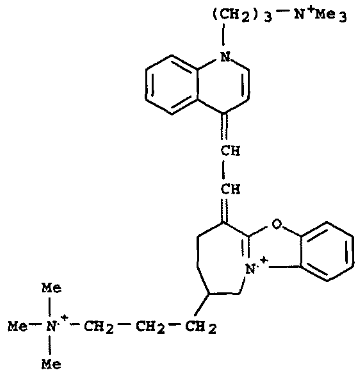

7,8,9,10-tétrahydro-9-[3-(triméthylammonio)propyl]-6-[[1-[3-(triméthylammonio)propyl]-4(1H)-quinolinylidene]éthylidene]-6H-azépino[2,1-b]benzoxazolium

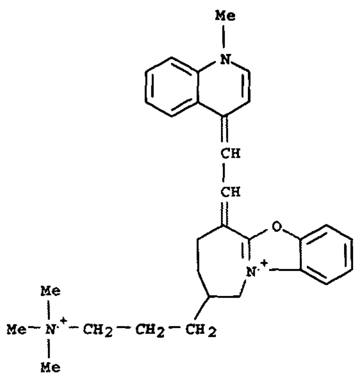

7,8,9,10-tétrahydro-6-[(1-méthyl-4(1H)-quinolinylidene)éthylidene]-9-[3-(triméthylammonio)propyl]-6H-azépino[2,1-b] benzoxazolium

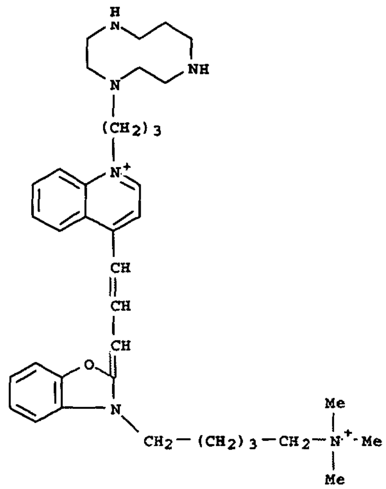

1-[3-(octahydro-1,4,7-triazecin-4(1H)-yl)propyl]-4-[3-[3-[5-(triméthylammonio)pentyl]-2(3H)-benzoxazolylidene]-1-propényl]-quinolinium

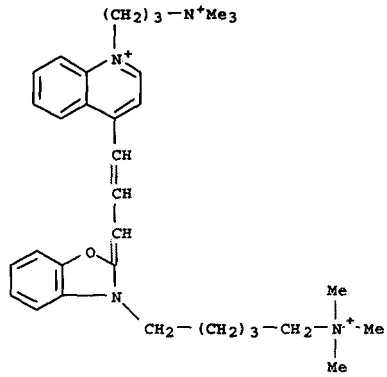

Quinolinium, 1-[3-(trimethylammonio)propyl]-4-[3-[3-[5-(trimethylammonio)pentyl]-2(3H)-benzoxazolylidene]-1-propenyl]-(9CI)

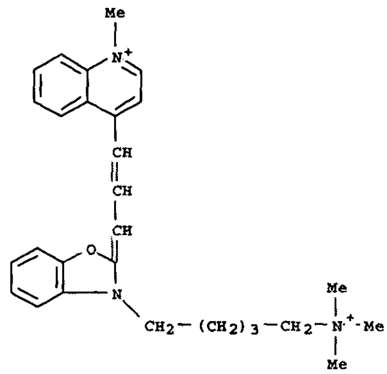

1-méthyl-4-[3-[3-[5-(triméthylammonio)pentyl]-2(3H)-benzoxazolylidene]-1-propényl]-quinolinium

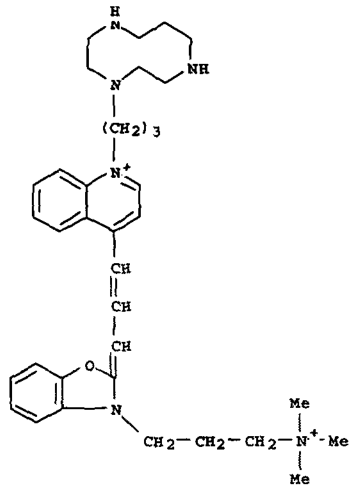

1-[3-(octahydro-1,4,7-triazecin-4(1H)-ylidene)propyl]-4-[3-[3-[3-(triméthylammonio)propyl]-2(3H)-benzoxazolylidene]-1-propényl]-quinolinium

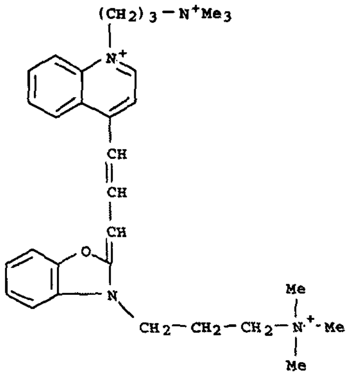

1-[3-(triméthylammonio) propyl]- 4-[3-[3-[3-(triméthylammonio) propyl]- 2 (3H)-benzoxazolylidene]- 1- propényl]-quinolinium

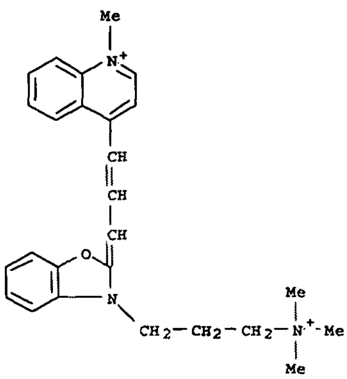

1-méthyl-4-[3-[3-[3-(triméthylammonio)propyl]-2(3H)-benzoxazolylidene]-1-propényl]-quinolinium

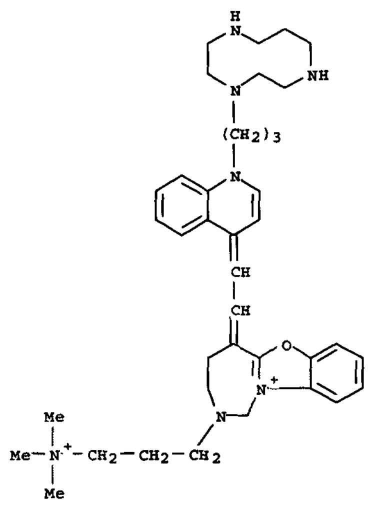

2,3,4,5-tétrahydro-5-[[1-[3-(octahydro-1,4,7-triazecin-4(1H)-yl)propyl]-4(1H)-quinolinylidene]éthylidene]-2-[3-(triméthylammonio)propyl]-1H-[1,3]diazépino[7,1-b]benzoxazol-11-ium

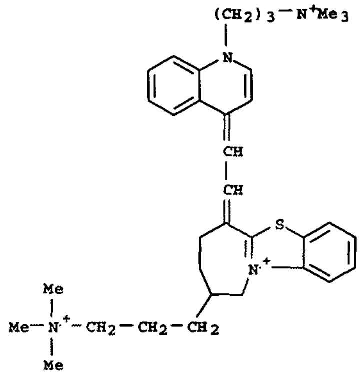

7,8,9,10-tétrahydro-9-[3-(triméthylammonio)propyl]-6-[[1-[3-(triméthylammonio)propyl]-4(1H)-quinolinylidene]éthylidene]-6H-azépino[2,1-b]benzothiazolium

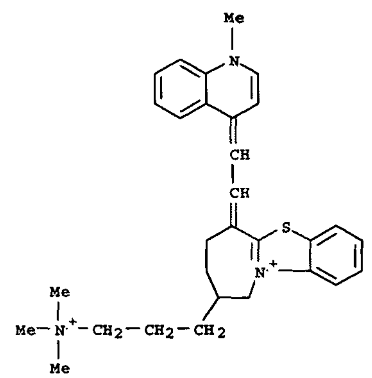

7,8,9,10-tétrahydro-6-[(1-méthyl-4(1H)-quinolinylidene)éthylidene]-9-[3-(triméthylammonio)propyl]-6H-azépino[2,1-b]benzothiazolium

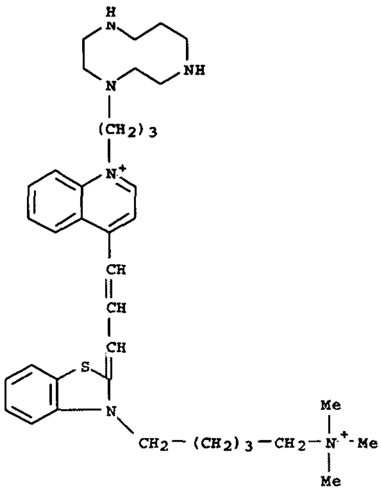

1-[3-(octahydro- 1,4,7- triazecin- 4 (1H)-yl) propyl]- 4-[3-[3-[5-(triméthylammonio) pentyl]- 2 (3H)-benzothiazolylidene]- 1-propényl]-quinolinium

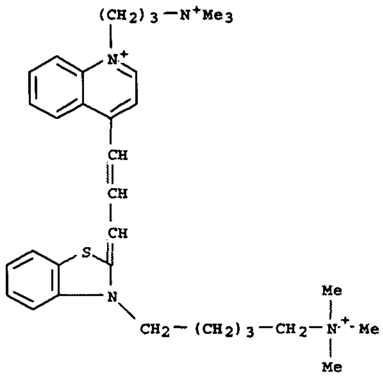

1-[3-(triméthylammonio) propyl]- 4-[3-[3-[5-(triméthylammonio) pentyl]- 2 (3H)-benzothiazolylidene]- 1- propényl]-quinolinium

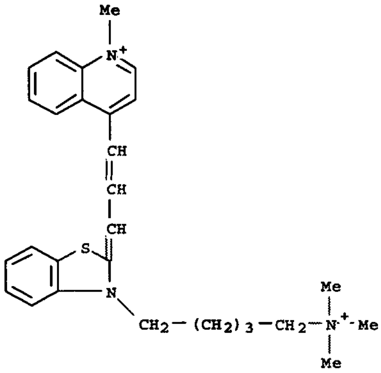

1-méthyl-4-[3-[3-[5-(triméthylammonio)pentyl]-2(3H)-benzothiazolylidene]-1-propényl]-quinolinium

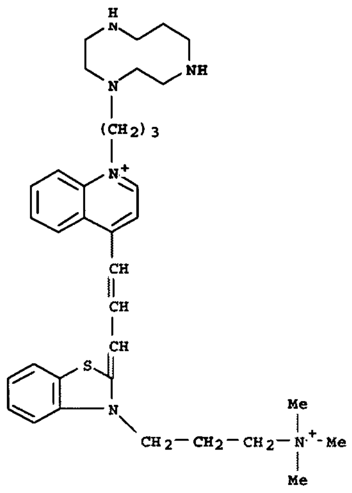

1-[3-(octahydro-1,4,7-triazecin-4(1H)-ylidene)propyl]-4-[3-[3-[3-(triméthylammonio)propyl]-2(3H)-benzothiazolylidene]-1-propényl]-quinolinium

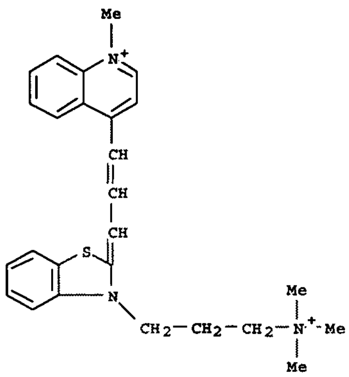

1-méthyl-4-[3-[3-[3-(triméthylammonio)propyl]-2(3H)-benzothiazolylidene]-1-propényl]-quinolinium

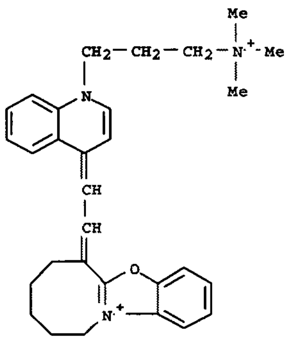

6,7,8,9,10,11-hexahydro-6-[[1-[3-(triméthylammonio)propyl]-4(1H)-quinolinylidene]éthylidene]-azocino[2,1-b]benzoxazolium

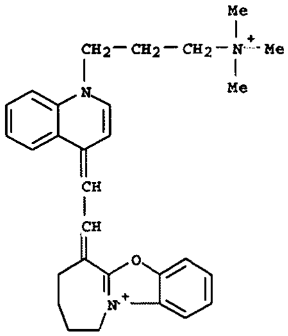

7,8,9,10-tétrahydro-6-[[1-[3-(triméthylammonio)propyl]-4(1H)-quinolinylidene]éthylidene]-6H-azépino[2,1-b]benzoxazolium

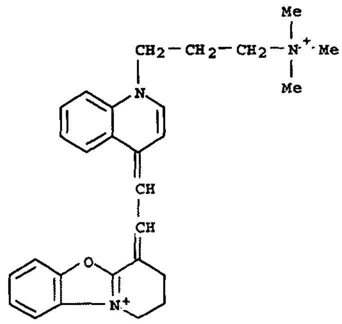

1,2,3,4-tétrahydro-4-[[1-[3-(triméthylammonio)propyl]-4(1H)-quinolinylidene]éthylidene]-pyrido[2,1-b]benzoxazolium

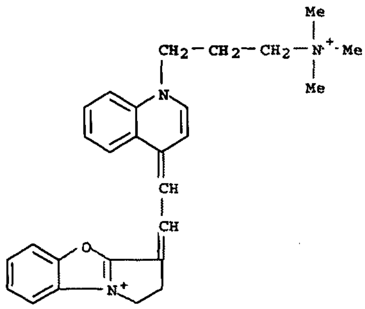

2,3-dihydro-3-[[1-[3-(triméthylammonio)propyl]-4(1H)-quinolinylidene]éthylidene]-1H-pyrrolo[2,1-b]benzoxazolium

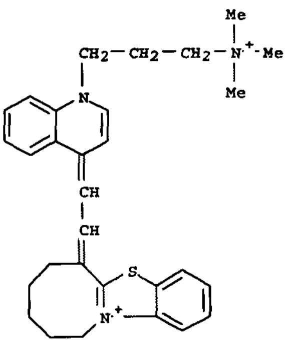

6,7,8,9,10,11-hexahydro-6-[[1-[3-(triméthylammonio)propyl]-4(1H)-quinolinylidene]éthylidene]-azocino[2,1-b]benzo-

thiazolium

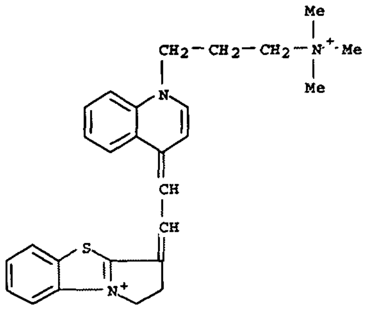

2,3-dihydro-3-[[1-[3-(triméthylammonio)propyl]-4(1H)-quinolinylidene]éthylidene]-1H-Pyrrolo[2,1-b]benzothiazolium

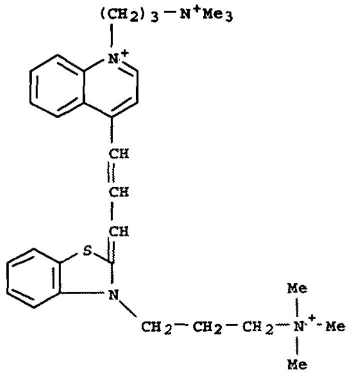

1-[3-(triméthylammonio) propyl]- 4-[3-[3-[3-(triméthylammonio) propyl]- 2 (3H)-benzothiazolylidene]- 1- propényl]-quinolinium

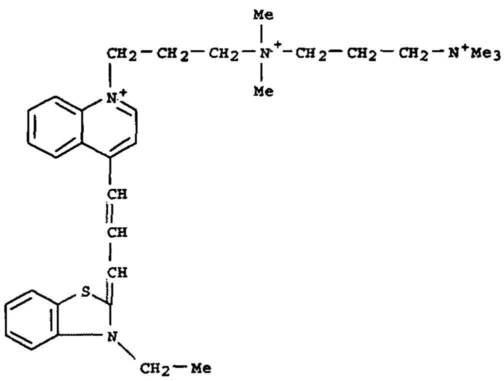

1-[3-[diméthyl[3-(triméthylammonio)propyl]ammonio]propyl]-4-[3-(3-éthyl-2(3H)-benzothiazolylidene)-1-propényl]-quinolinium

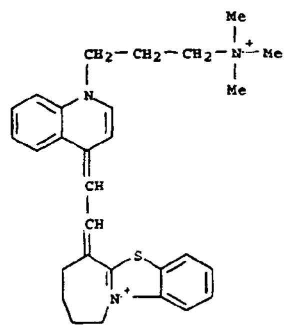

7,8,9,10-tétrahydro-6-[[1-[3-(triméthylammonio)propyl]-4(1H)-quinolinylidene]éthylidene]-6H-azépino[2,1-b]benzothiazolium

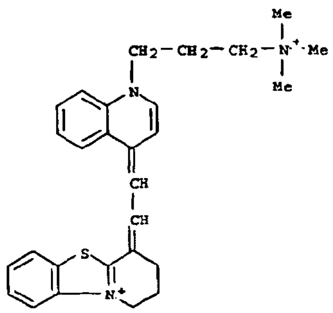

1,2,3,4-tétrahydro-4-[[1-[3-(triméthylammonio)propyl]-4(1H)-quinolinylidene]éthylidene]-pyrido[2,1-b]benzothiazolium

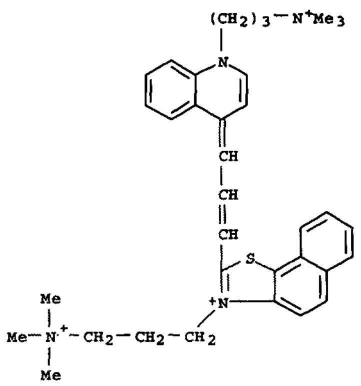

3-[3-(triméthylammonio)propyl]-2-[3-[1-[3-(triméthylammonio)propyl]-4(1H)-quinolinylidene]-1-propényl]-naphtho[2,1-d]thiazolium

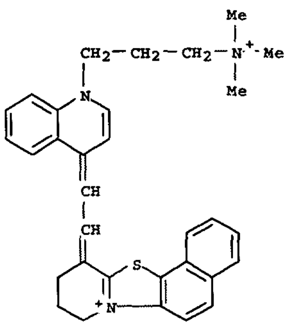

8,9,10,11-tétrahydro-11-[[1-[3-(triméthylammonio)propyl]-4(1H)-quinolinylidene]éthylidene]-naphtho[2',1':4,5]thiazolo [3,2-a]pyridinium

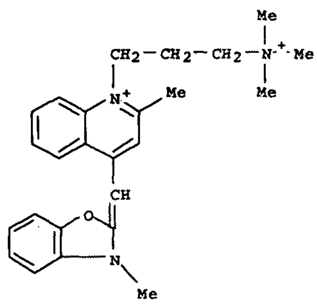

$\cdot\ 2\ I^-$

diiodure de 2-méthyl-4-[(3-méthyl-2(3H)-benzoxazolylidene)méthyl]-1-[3-(triméthylammonio)propyl]-quinolinium

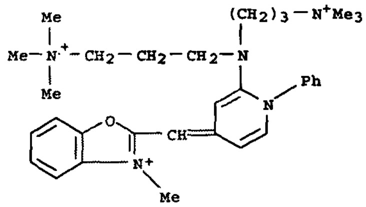

2-[[2-[bis[3-(triméthylammonio)propyl]amino]-1-phényl-4(1H)-pyridinylidene]méthyl]-3-méthyl-benzoxazolium

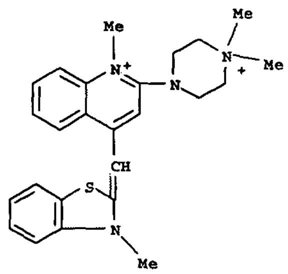

2-(4,4-diméthylpipérazinium-1-yl)-1-méthyl-4-[(3-méthyl-2(3 H)-benzothiazolylidene)méthyl]-quinolinium

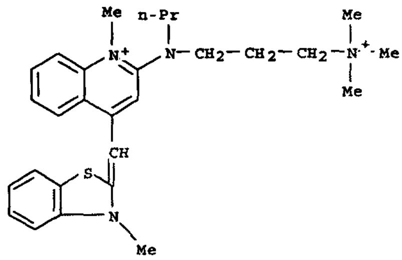

1-méthyl-4-[(3-méthyl-2(3H)-benzothiazolylidene)méthyl]-2-[propyl[3-(triméthylammonio)propyl]amino]-quinolinium

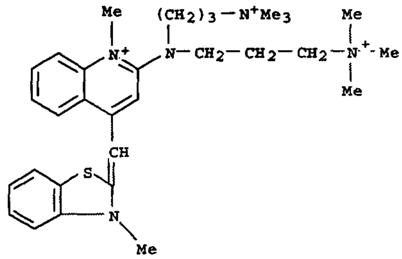

2-[bis[3-(triméthylammonio)propyl]amino]-1-méthyl-4-[(3-méthyl-2(3H)-benzothiazolylidene)méthyl]-quinolinium

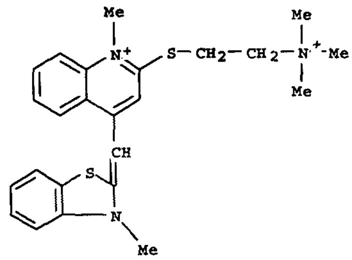

1-méthyl-4-[(3-méthyl-2(3H)-benzothiazolylidene)méthyl]-2-[[2-(triméthylammonio)éthyl]thio]-quinolinium

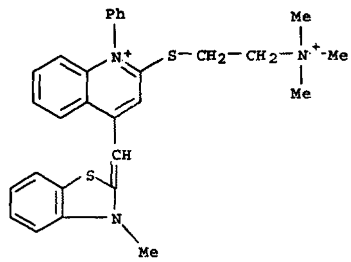

4-[(3-méthyl-2(3H)-benzothiazolylidene)méthyl]-1-phényl-2-[[2-(triméthylammonio)éthyl]thio]-quinolinium

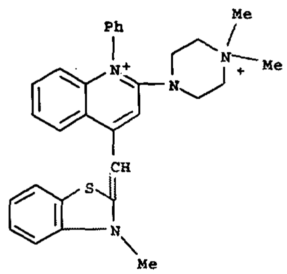

2-(4,4-diméthylpipérazinium-1-yl)-4-[(3-méthyl-2(3H)-benzothiazolylidene)méthyl]-1-phényl-quinolinium

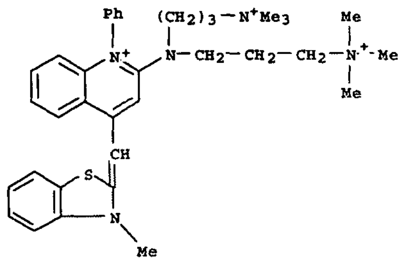

2-[bis[3-(triméthylammonio)propyl]amino]-4-[(3-méthyl-2(3H)-benzothiazolylidene)méthyl]-1-phényl-quinolinium

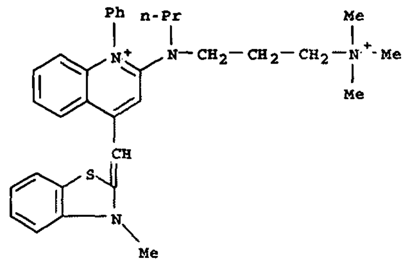

· 2 $I^-$

4-[(3-méthyl-2(3H)-benzothiazolylidene)méthyl]-1-phényl-2-[propyl[3-(triméthylammonio)propyl]amino]-quinolinium

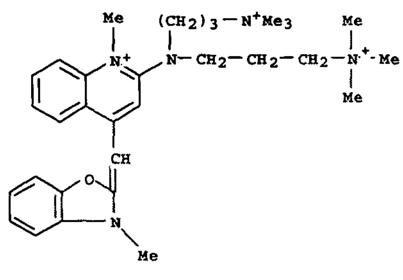

2-[bis[3-(triméthylammonio)propyl]amino]-1-méthyl-4-[(3-méthyl-2(3H)-benzoxazolylidene)méthyl]-quinolinium

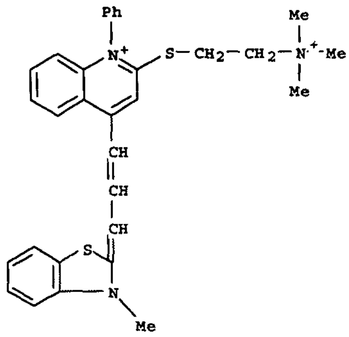

4-[3-(3-méthyl-2(3H)-benzothiazolylidene)-1-propényl]-1-phényl-2-[[2-(triméthylammonio)éthyl]thio]-quinolinium

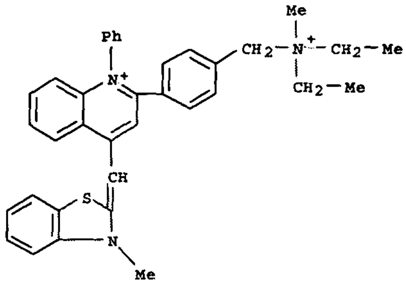

2-[4-[(diéthylméthylammonio)méthyl]phényl]-4-[(3-méthyl-2(3H)-benzothiazolylidene)méthyl]-1-phényl-quinolinium

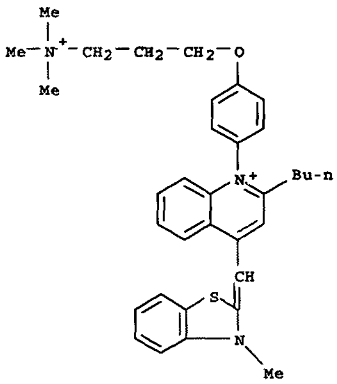

2-butyl-4-[(3-méthyl-2(3H)-benzothiazolylidene)méthyl]-1-[4-[3-(triméthylammonio)propoxy]phényl]-quinolinium

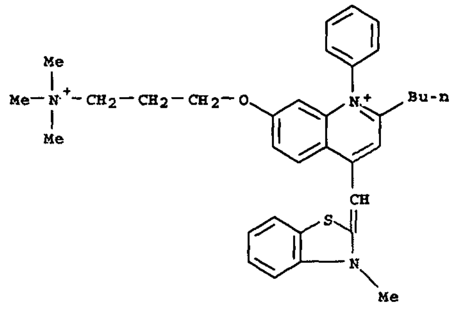

2-butyl-4-[(3 -méthyl-2(3 H)-benzothiazolylidene)méthyl]-1-phényl-7-[3-(triméthylammonio)propoxy]-quinolinium

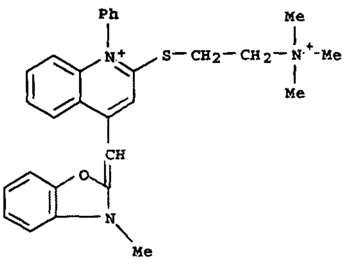

4-[(3-méthyl-2(3H)-benzoxazolylidene)méthyl]-1-phényl-2-[[2-(triméthylammonio)éthyl]thio]-quinolinium

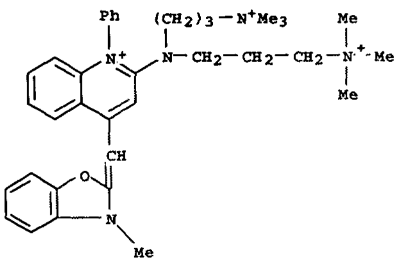

2-[bis[3-(triméthylammonio)propyl]amino]-4-[(3-méthyl-2(3H)-benzoxazolylidene)méthyl]-1-phényl-quinolinium

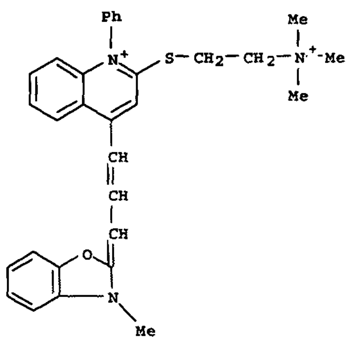

4-[3-(3-méthyl-2(3H)-benzoxazolylidene)-1-propényl]-1-phényl-2-[[2-(triméthylammonio)éthyl]thio]-quinolinium

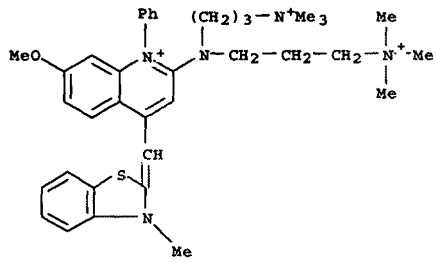

2-[bis[3-(triméthylammonio)propyl]amino]-7-méthoxy-4-[(3-méthyl-2(3H)-benzothiazolylidene)méthyl]-1-phényl-quinolinium

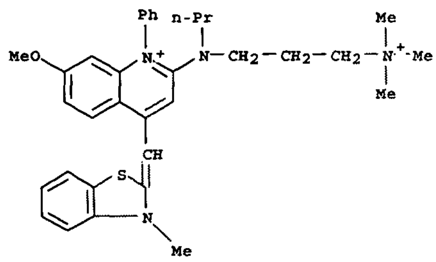

7-methoxy-4-[(3-methyl-2(3 H)-benzothiazolylidene)methyl]-1-phenyl-2-[propyl[3-(trimethylammonio)propyl]amino]-quinolinium

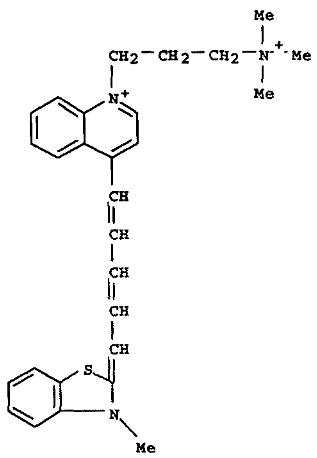

$\cdot 2\ I^-$

Quinolinium, 4-[5-(3-méthyl-2(3H)-benzothiazolylidene)-1,3-pentadienyl]-1-[3-(triméthylammonio)propyl]-, diiodide (9Cl) (diiodure de 3-méthyl-2-[5-[1-[3-(triméthylammonio)propyl]-4(1H)-quinolinylidene]-1,3-pentadienyl]benzothiazolium)

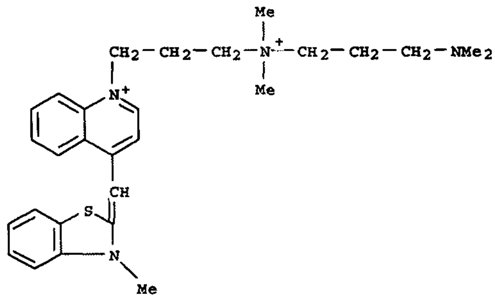

$\cdot 2\ I^-$

$\cdot HI$

diiodure de 1-[3-[[3-(dimethylamino)propyl]dimethylammonio]propyl] -4-[(3-methyl-2(3H)-benzothiazolylidene) methyl]-quinolinium, monohydriodure

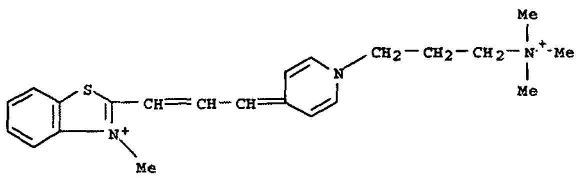

$\cdot 2\ I^-$

diiodure de 3-méthyl-2-[3-[1-[3-(triméthylammonio)propyl]-4(1H)-pyridinylidene]-1-propényl]-benzothiazolium

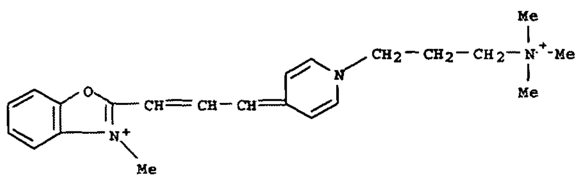

$\cdot 2\ I^-$

diiodure de 3-méthyl-2-[3-[1-[3-(triméthylammonio)propyl]-4(1H)-pyridinylidene]-1-propényl]-benzoxazolium

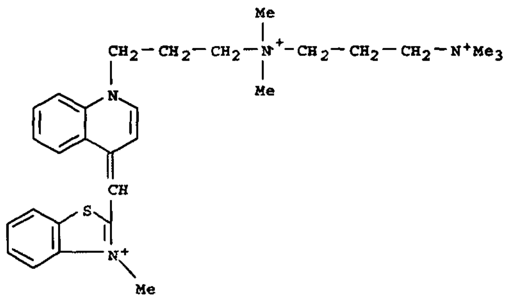

· 3 $I^-$

triiodure de 2-[[1-[3-[diméthyl[3-(triméthylammonio)propyl]-ammonio]propyl]-4(1H)-quinolinylidene]méthyl]-3-méthyl-benzothiazolium

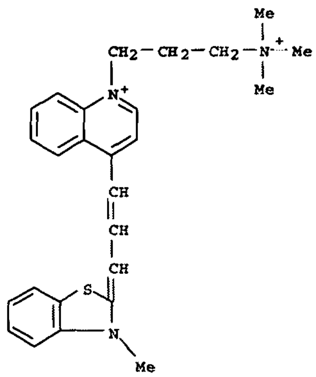

· 2 $I^-$

diiodure de 4-[3-(3-méthyl-2(3H)-benzothiazolylidene)-1-propényl]-1-[3-(triméthylammonio)propyl]-quinolinium

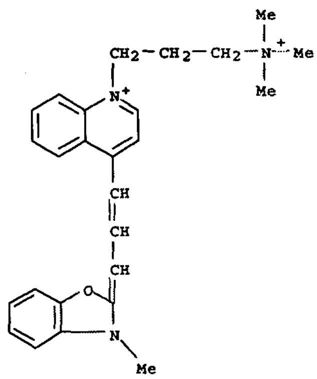

· 2 $I^-$

diiodure de 4-[3-(3-méthyl-2(3H)-benzoxazolylidene)-1-propényl]-1-[3-(triméthylammonio)propyl]-quinolinium

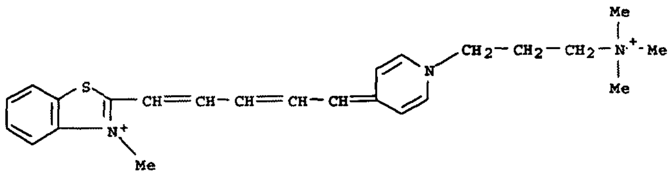

$\cdot 2\ I^-$

diiodure de 3-méthyl-2-[5-[1-[3-(triméthylammonio)propyl]-4(1H)-pyridinylidene]-1,3-pentadienyl]-benzothiazolium

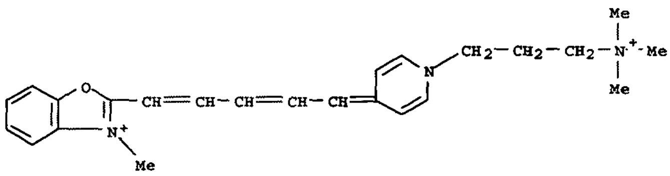

$\cdot 2\ I^-$

diiodure de 3-méthyl-2-[5-[1-[3-(triméthylammonio)propyl]-4(1H)-pyridinylidene]-1,3-pentadienyl]-benzoxazolium

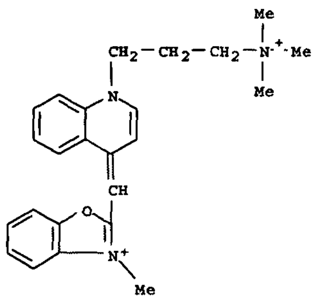

$\cdot 2\ I^-$

diiodure de 3-méthyl-2-[[1-[3-(triméthylammonio)propyl]-4(1H)-quinolinylidene]méthyl]-benzoxazolium

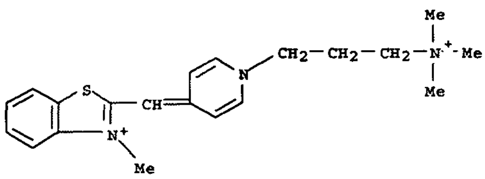

$\cdot 2\ I^-$

diiodure de 3-méthyl-2-[[1-[3-(triméthylammonio)propyl]-4(1H)-pyridinylidene]méthyl]-benzothiazolium

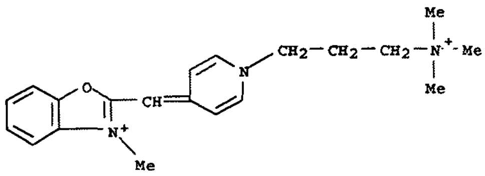

$\cdot 2\ I^-$

diiodure de 3-méthyl-2-[[1-[3-(triméthylammonio)propyl]-4(1H)-pyridinylidene]méthyl]-benzoxazolium

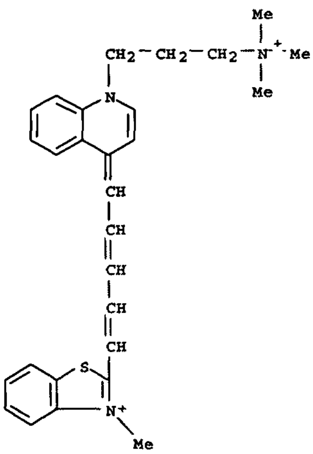

3-méthyl-2-[5-[1-[3-(triméthylammonio)propyl]-4(1H)-quinolinylidene]-1,3-pentadiényl]-benzothiazolium

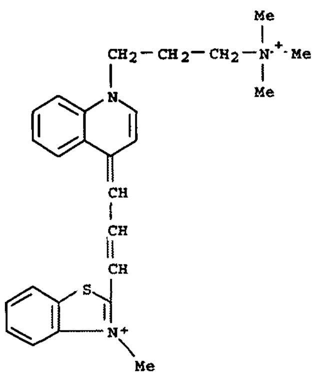

3-méthyl-2-[3-[1-[3-(triméthylammonio)propyl]-4(1H)-quinolinylidene]-1-propényl]-benzothiazolium

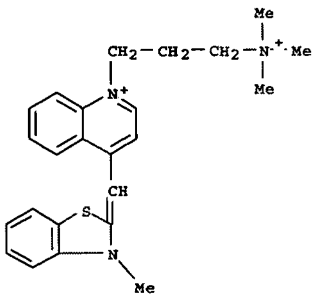

4-[(3-méthyl-2(3H)-benzothiazolylidene)méthyl]-1-[3-(triméthylammonio)propyl]-quinolinium

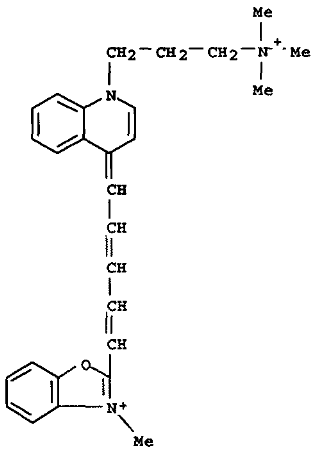

3-méthyl-2-[5-[1-[3-(triméthylammonio)propyl]-4(1H)-quinolinylidene]-1,3-pentadiényl]-benzoxazolium

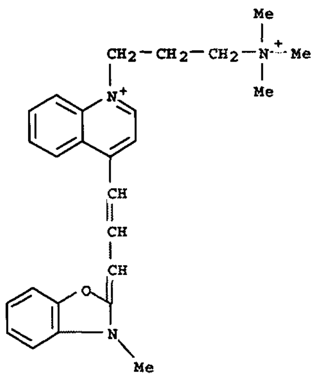

4-[3-(3-méthyl-2(3H)-benzoxazolylidene)-1-propényl]-1-[3-(triméthylammonio)propyl]-quinolinium

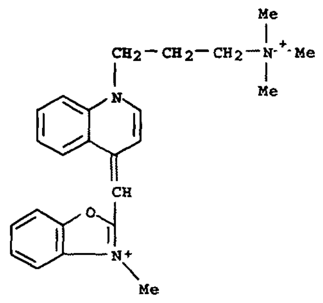

3-méthyl-2-[[1-[3-(triméthylammonio)propyl]-4(1H)-quinolinylidene]méthyl]-benzoxazolium

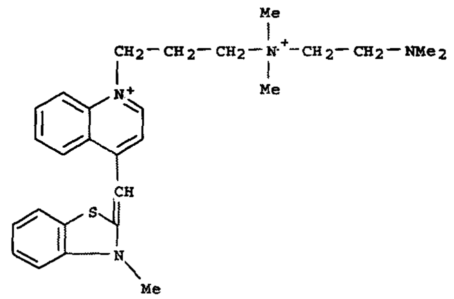

1-[3-[[2-(diméthylamino)éthyl]diméthylammonio]propyl]-4-[(3-méthyl-2(3H)-benzothiazolylidene)méthyl]-quinolinium

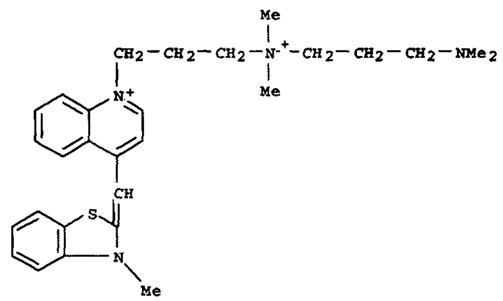

1-[3-[[3-(diméthylamino)propyl]diméthylammonio]propyl]-4-[(3-méthyl-2(3H)-benzothiazolylidene)méthyl]-quinolinium

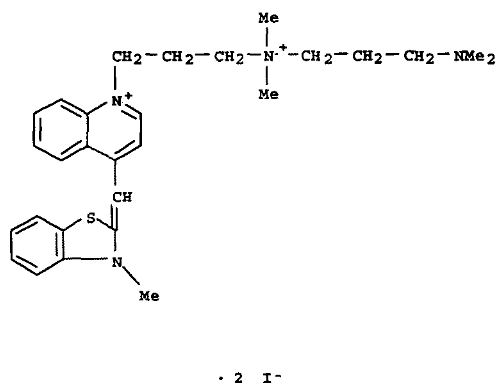

diiodure de 1-[3-[[3-(diméthylamino)propyl]diméthylammonio]-propyl]-4-[(3-méthyl-2(3H)-benzothiazolylidene)méthyl]-quinolinium

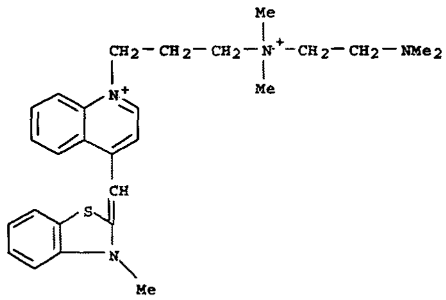

$\cdot 2\ I^-$

diiodure de 1-[3-[[2-(diméthylamino)ethyl]diméthylammonio]propyl]-4-[(3-méthyl-2(3H)-benzothiazolylidene)méthyl]-quinolinium

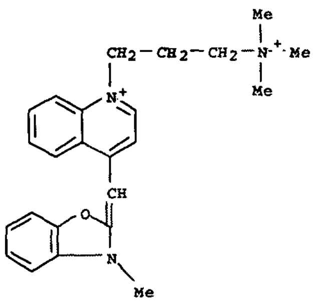

$\cdot 2\ I^-$

diiodure de 4-[(3-méthyl-2(3H)-benzoxazolylidene)méthyl]-1-[3-(triméthylammonio)propyl]-quinolinium

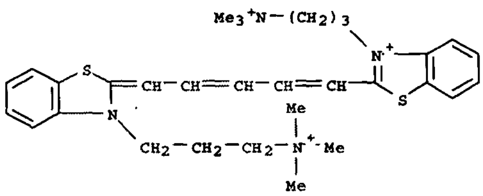

$\cdot 3\ Br^-$

tribromure de 3-[3-(triméthylammonio)propyl]-2-[5-[3-[3-(triméthylammonio)propyl]-2(3H)-benzothiazolylidene]-1,3-pentadienyl]-benzothiazolium

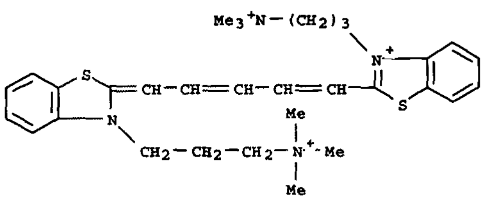

3-[3-(triméthylammonio)propyl]-2-[5-[3-[3-(triméthylammonio)propyl]-2(3H)-benzothiazolylidene]-1,3-pentadienyl]-benzothiazolium

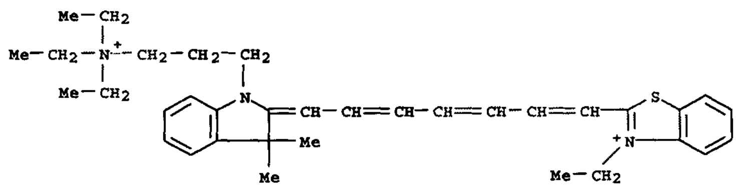

$\cdot 2\ Br^-$

dibromure de 2-[7-[1,3-dihydro-3,3-diméthyl-1-[3-(triéthylammonio)-propyl]-2H-indol-2-ylidene]-1,3,5-heptatrienyl]-3-éthyl-benzothiazolium

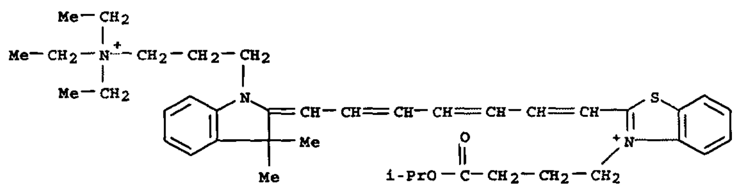

$\cdot 2\ Br^-$

dibromure de 2-[7-[1,3-dihydro-3,3-diméthyl-1-[3-(triéthylammonio)-propyl]-2H-indol-2-ylidene]-1,3,5-heptatrienyl]-3-[4-(1-méthyléthoxy)-4-oxobutyl]-benzothiazolium

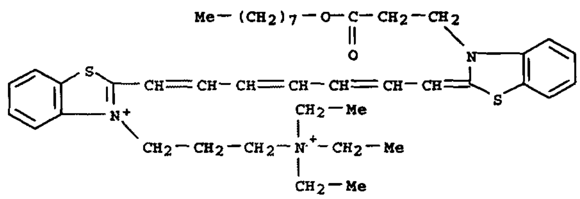

$\cdot 2\ Br^-$

dibromure de 2-[7-[3-[3-(octyloxy)-3-oxopropyl]-2(3H)-benzothiazolylidene]-1,3,5-heptatrienyl]-3-[3-(triéthylammonio) propyl]-benzothiazolium

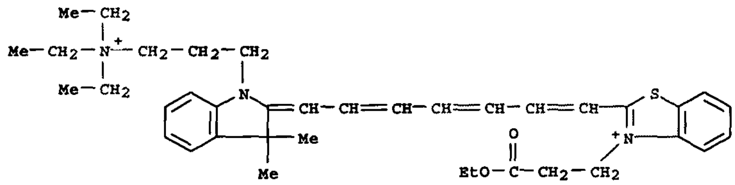

$\cdot 2\ Br^-$

dibromure de 2-[7-[1,3-dihydro-3,3-diméthyl-1-[3-(triéthylammonio)-propyl]-2H-indol-2-ylidene]-1,3,5-heptatrienyl]-3-(3-éthoxy-3-oxopropyl)-benzothiazolium

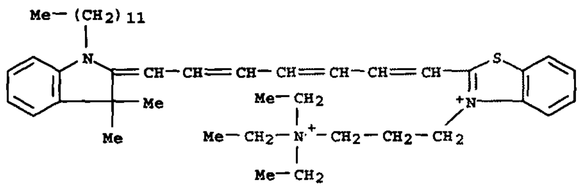

· 2 $Br^-$

dibromure de 2-[7-(1-dodécyl-1,3-dihydro-3,3-diméthyl-2H-indol-2-ylidene)-1,3,5-heptatrienyl]-3-[3-(triéthylammonio) propyl]-benzothiazolium

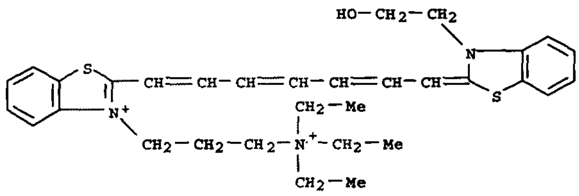

· 2 $Br^-$

dibromure de 2-[7-[3-(2-hydroxyéthyl)-2(3H)-benzothiazolylidene]-1,3,5-heptatrienyl]-3-[3-(triéthylammonio)propyl]-benzothiazolium

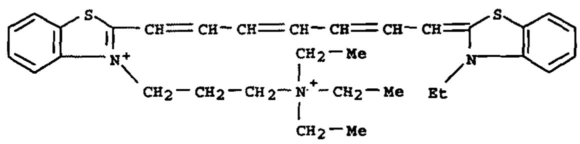

· 2 $Br^-$

dibromure de 2-[7-(3-éthyl-2(3H)-benzothiazolylidene)-1,3,5-heptatrienyl]-3-[3-(triéthylammonio)propyl]-benzothiazolium

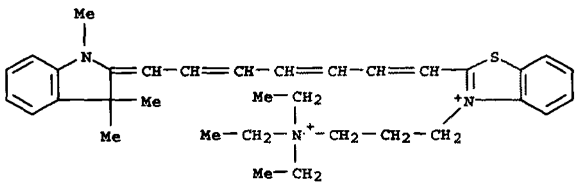

· 2 $Br^-$

dibromure de 2-[7-(1,3-dihydro-1,3,3-triméthyl-2H-indol-2-ylidene)-1,3,5-heptatrienyl]-3-[3-(triéthylammonio)pro-

pyl]-benzothiazolium

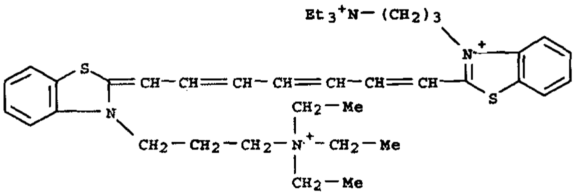

$F_3C-CF_2-CF_2-CO_2^-$

Benzothiazolium, 3-[3-(triéthylammonio)propyl]-2-[7-[3-[3-(triéthylammonio)propyl]-2(3H)-benzothiazolylidene]-1,3,5-heptatriényl]-, sel avec de l'acide heptafluorobutanoique (1:3) (9CI) (Acide Butanoique, heptafluoro-, ion(1-),3-[3-(triéthylammonio) propyl]- 2-[7-[3-[3-(triéthylammonio) propyl]- 2 (3H)-benzothiazolylidene]- 1,3,5- heptatriényl] benzothiazolium)

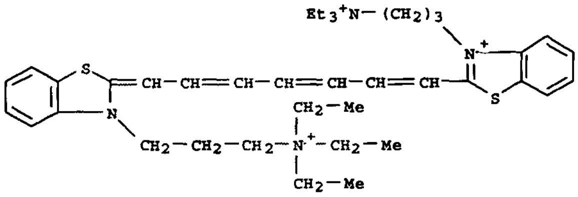

3-[3-(triéthylammonio) propyl]- 2-[7-[3-[3-(triéthylammonio) propyl]- 2 (3H)-benzothiazolylidene]- 1,3,5- heptatriényl]-benzothiazolium

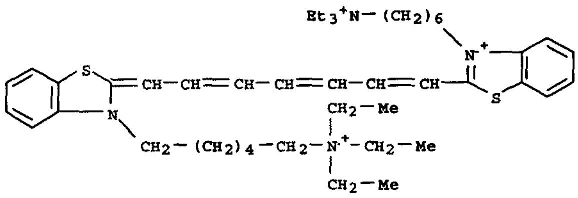

· 3 $Br^-$

tribromure de 3-[6-(triéthylammonio)hexyl]-2-[7-[3-[6-(triéthylammonio)hexyl]-2(3H)-benzothiazolylidene]-1,3,5-heptatrienyl]-benzothiazolium

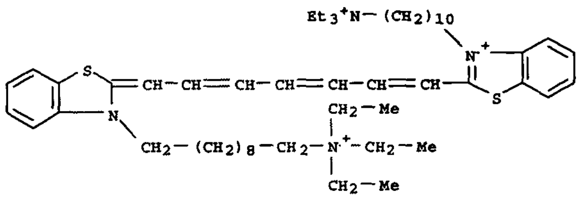

· 3 $Br^-$

tribromure de 3-[10-(triéthylammonio)décyl]-2-[7-[3-[10-(tri éthylammo ni o)décyl]-2(3H)-benzothiazolylid en e]- 1,3,5-heptatriényl]-benzothiazolium

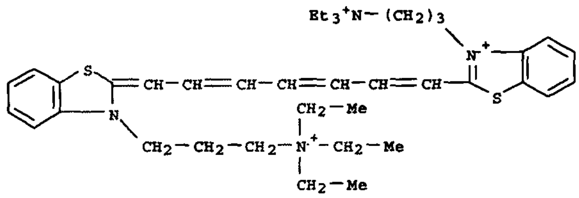

· 3 $Br^-$

tribromure de 3-[3-(triéthylammonio)propyl]-2-[7-[3-[3-(triethylammonio)propyl]-2(3H)-benzothiazolylidene]-1,3,5-heptatriényl]-benzothiazolium

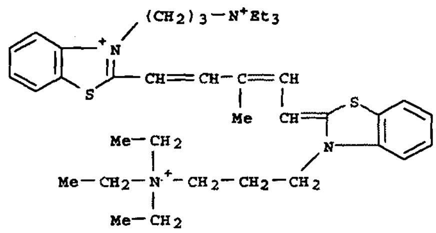

· 3 $Br^-$

tribromure de 2-[3-méthyl-5-[3-[3-(triéthylammonio)propyl]-2(3H)-benzothiazolylidene]-1,3-pentadienyl]-3 -[3-(triéthylammonio)propyl]-benzothiazolium

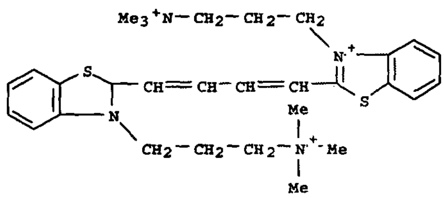

· 3 $Br^-$

tribromure de 2-[4-[2,3-dihydro-3-[3-(triméthylammonio)propyl]-2-benzothiazolyl]-1,3-butadienyl]-3-[3-(triméthylammonio)propyl]-benzothiazolium

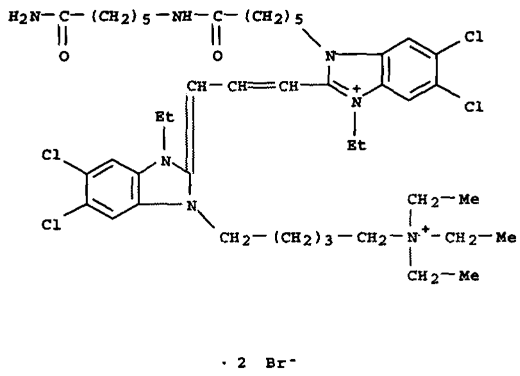

dibromure de 1-[6-[(6-amino-6-oxohexyl)amino]-6-oxohexyl]-5,6-dichloro-2-[3-[5,6-dichloro-1-éth yl-1,3-dih ydro-3-[5-(triéthylammonio)pentyl]-2H-benzimidazol-2-ylidene]-1-propényl]-3-éthyl-1H-benzimidazolium

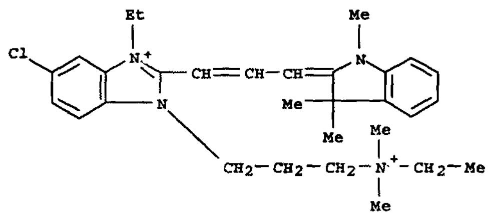

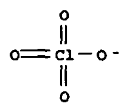

perchlorate de 5-chloro-1-(3-diméthylaminopropyl)-3-éthyl-2,3-dihydro-2-[3-(1,3,3-triméthyl-2-indolinylidene)propényl]-2-benzimidazolocarbonium perchlorate

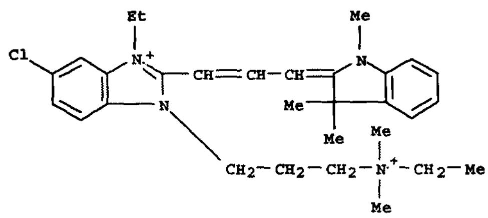

5-chloro-2-[3-(1,3-dihydro-1,3,3-triméthyl-2H-indol-2-ylidene)-1-propényl]-3-éthyl-1-[3-(éthyldiméthylammonio)propyl]-1H-benzimidazolium

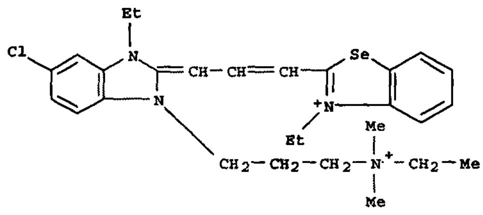

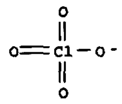

erchlorate de 5-chloro-1-(3-diméthylaminopropyl)-3-éthyl-2-[3-(3-éthyl-2-benzoselenazolinylidene)propényl]-2,3-dihydro-2-benzimidazolocarbonium (6Cl)

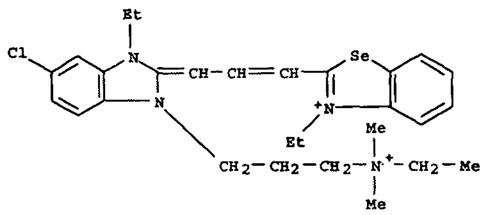

2-[3-[5- chloro- 3-éthyl- 1-[3-(éthyldimethylammonio) propyl]- 1,3- dihydro- 2H- benzimidazol- 2- ylidene]- 1- propényl]-3-éthyl-benzoselenazolium

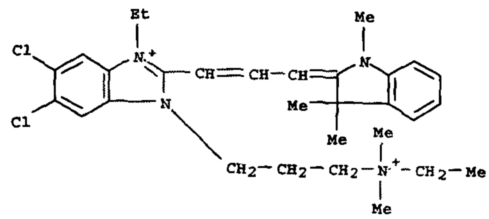

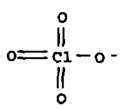

5,6-dichloro-1-(3-diméthylaminopropyl)-3-éthyl-2,3-dihydro-2-[3-(1,3,3-triméthyl-2-indolinylidene)propényl]-2-benzimidazolocarbonium perchlorate éthoperchlorate (6Cl) (perchlorate de 2-[3-[5,6-dichloro-1-(3-diméthylaminopropyl)-3-éthyl-2-benzimidazolinylidene]propényl]-2,3-dihydro-1,3,3-triméthyl-2-indolocarbonium)

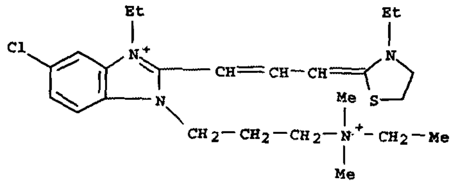

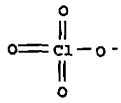

perchlorate de 5-chloro-1-(3-dimethylaminopropyl)-3-éthyl-2-(3-(3-éthyl-2-thiazolidinylidene)propényl]-2,3-dihydro-2-benzimidazolocarbonium (6CI)

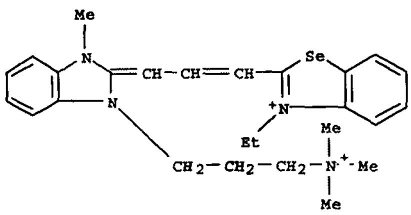

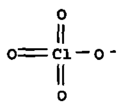

perchlorate de 1-(3-miméthylaminopropyl)-2-[3-(3-éthyl-2-benzoselenazolinylidene)propényl]-2,3-dihydro-3-méthyl-2-benzimidazolocarbonium perchlorate (6CI)

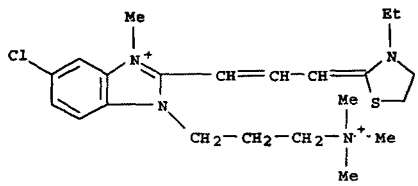

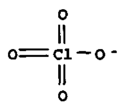

perchlorate de 5-chloro-1-(3-diméthylaminopropyl)-2-[3-(3-éthyl-2-thiazolidinylidene)propényl]-2,3-dihydro-3-méthyl-2-benzimidazolocarbonium perchlorate (6CI)

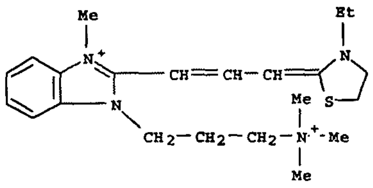

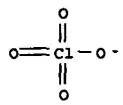

perchlorate de 1-(3-diméthylaminopropyl)-2-[3-(3-éthyl-2-thiazolidinylidene)propényl]-2,3-dihydro-3-méthyl-2-benzimidazolocarbonium (6CI)

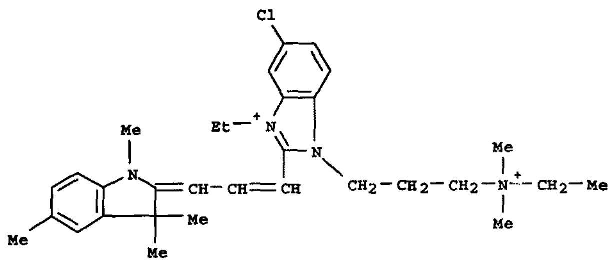

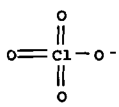

perchlorate de 2-[3-[5-chloro-1-(3-diméthylaminopropyl)-3-éthyl-2-benzimidazolinylidene]propényl]-2,3-dihydro-1,3,3,5-tetraméthyl-2-indolocarbonium (6CI)

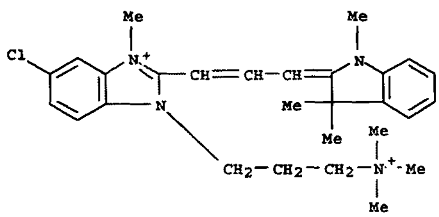

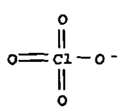

1H-Benzimidazolium, 5-chloro-2-[3-(1,3-dihydro-1,3,3-triméthyl-2H-indol-2-ylidene)-1-propényl]-3-méthyl-1-[3-(trimethylammonio)propyl]-, diperchlorate (9CI)
(perchlorate de 2-[3-[5-chloro-1-(3-diméthylaminopropyl)-3-méthyl-2-benzimidazolinylidene]propényl]-2,3-dihydro-1,3,3-triméthyl-2-indolocarbonium)

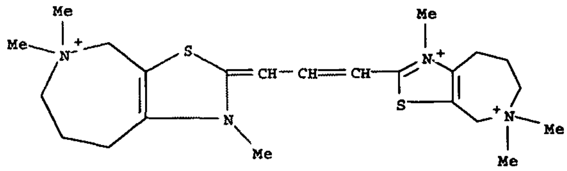

· 3 $I^-$

triiodure de 5,6,7,8-tétrahydro-1,5,5-triméthyl-2-[3-(5,6,7,8-tétrahydro-1,5,5-triméthyl-4H-thiazolo[5,4-c]azépin-2(1H)-ylidene)propényl]-4H-thiazolo[5,4-c]azépinediium

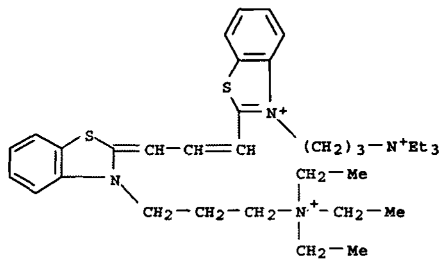

· 3 $Br^-$

tribromure de 3-[3-(triéthylammonio)propyl]-2-[3-[3-[3-(triéthylammonio)propyl]-2(3H)-benzothiazolylidene]-1-propényl]-benzothiazolium

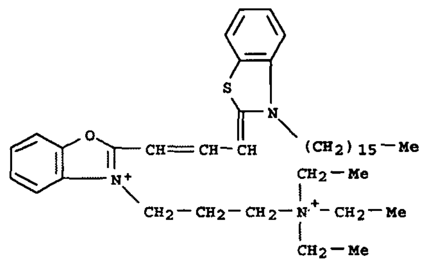

2 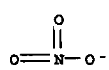

dinitrate de 2-[3-(3-hexadécyl-2(3H)-benzothiazolylidene)-1-propényl]-3-[3-(triéthylammonio)propyl]-benzoxazolium

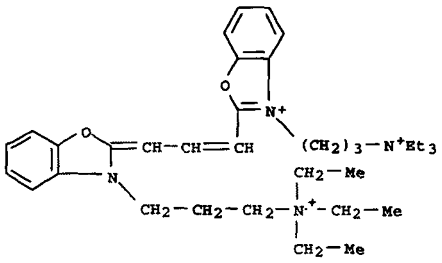

$\cdot 3\ Br^-$

tribromure de 3-[3-(triéthylammonio)propyl]-2-[3-[3-[3-(triéthylammonio)propyl]-2(3H)-benzoxazolylidene]-1-propenyl]-benzoxazolium

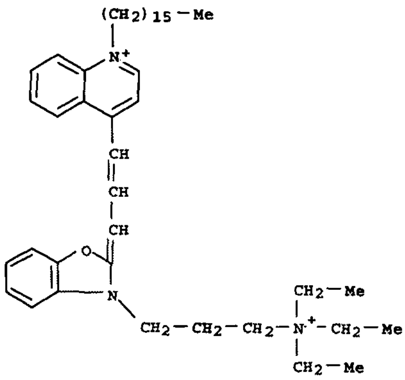

$\cdot 2\ Br^-$

dibromure de 1-hexadécyl-4-[3-[3-[3-(triéthylammonio)propyl]-2(3H)-benzoxazolylidene]-1-propényl]-quinolinium

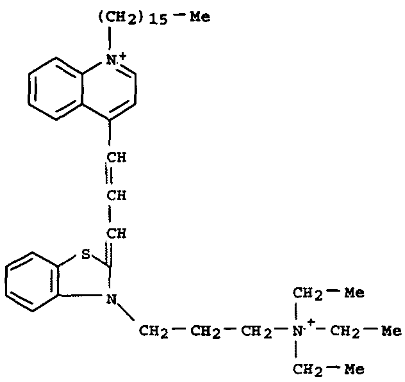

$\cdot 2\ Br^-$

dibromure de 1-hexadécyl-4-[3-[3-[3-(triéthylammonio)propyl]-2(3H)-benzothiazolylidene]-1-propényl]-quinolinium

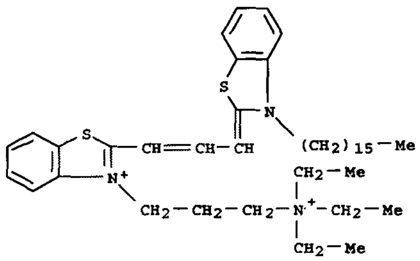

2 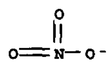

dinitrate de 2-[3-(3-hexadécyl-2(3H)-benzothiazolylidene)-1-propényl]-3-[3-(triéthylammonio)propyl]-benzothiazolium

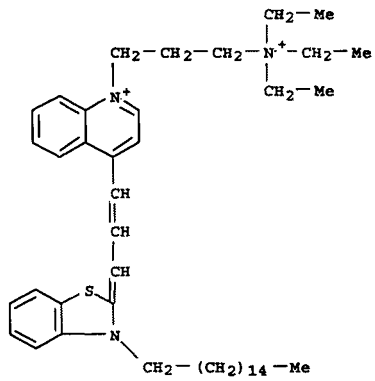

2 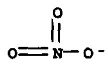

dinitrate de 4-[3-(3-hexadécyl-2(3H)-benzothiazolylidene)-1-propényl]-1-[3-(triéthylammonio)propyl]-quinolinium

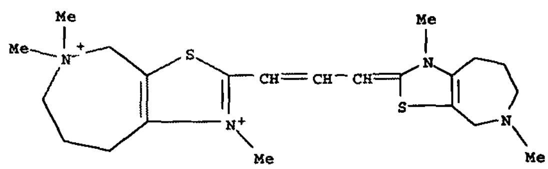

$\cdot 2\ I^-$

diiodure de -[3-(1,4,5,6,7,8-hexahydro-1,5-diméthyl-2H-thiazolo[5,4-c]azépin-2-ylidene)propényl]-5,6,7,8-tétrahydro-1,5,5-triméthyl-4H-thiazolo[5,4-c]azepinium

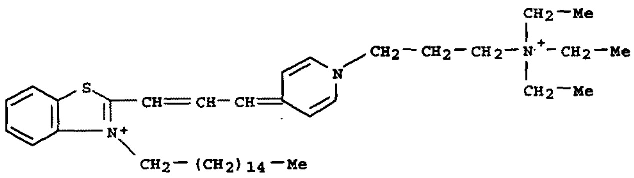

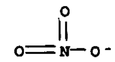

2

dinitrate de 3-hexadecyl-2-[3-[1-[3-(triethylammonio)propyl]-4(1H)-pyridinylidene]-1-propenyl]-benzothiazolium

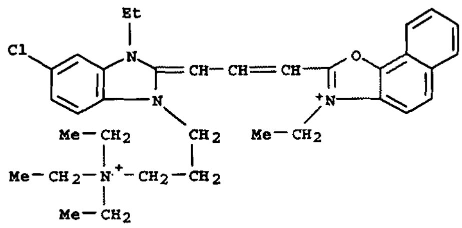

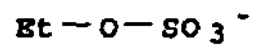

Naphth[2,1-d]oxazolium, 2-[3-[5-chloro-3-éthyl-1,3-dihydro-1-[3-(triÉthylammonio)propyl]-2H-benzimidazol-2-ylidene]-1-propényl]-3-Éthyl-, bis(Éthyl sulfate)
(Acide sulfurique, monoéthyl ester, on(1-),2-[3-[5-chloro-3-éthyl-1,3-dihydro-1-[3-(triéthylammonio)propyl]-2H-benzimidazol-2-ylidene]-1-propényl]-3-éthylnaphth[2,1-d]oxazolium (2:1))

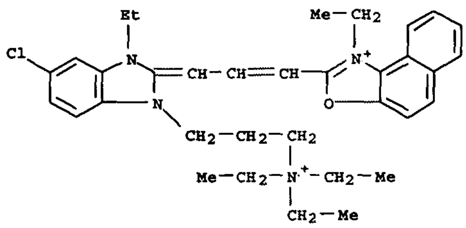

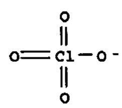

2

diperchlorate de 2-[3-[5-chloro-3-éthyl-1,3-dihydro-1-[3-(triéthylammonio)propyl]-2H-benzimidazol-2-ylidene]-1-propényl]-1-éthyl-naphth[1,2-d]oxazolium

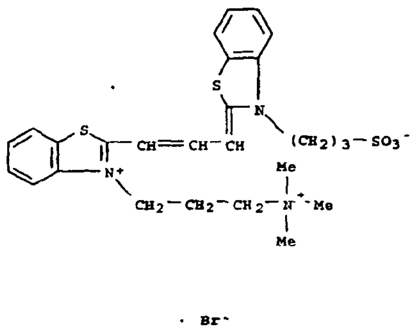

bromure de 2-[3-[3-(3-sutfopropyl)-2(3H)-benzothiazolylidene]-1-propenyl]-3-(3-(trimethylammonio)propyl)-benzothiazolium

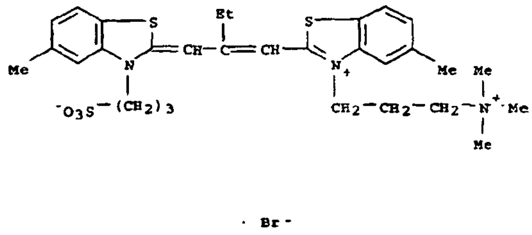

bromure de 5-méthyl-2-[2-[[5-méthyl-3-(3-sulfopropyl)-2(3H)-benzothiazolylidene]méthyl]-1-butényl]-3 -[3-(triméthylammonio)propyl]-benzothiazolium

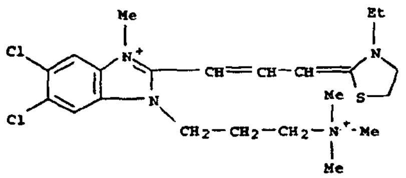

5,6-dichloro-2-[3-(3-ethyl-2-thiazolidinylidene)-1-propényl]-1-méthyl-3-[3-(triméthylammonio)propyl]-1H-benzimidazolium

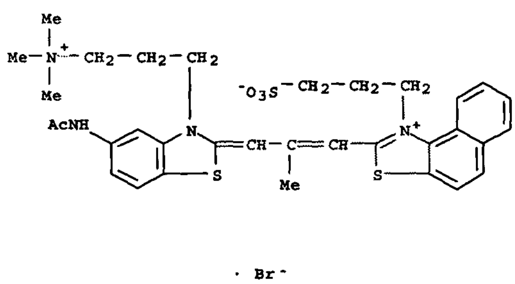

bromure de 2-[3-[5-(acétylamino)-3-[3-(triméthylammonio)propyl]-2(3H)-benzothiazolylidene]-2-méthyl-1-propényl]-1-(3-sulfopropyl)-naphtho[1,2-d]thiazolium

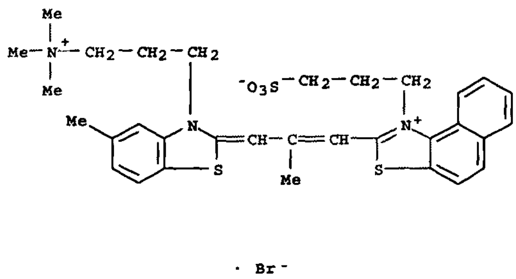

bromure de 2-[2-méthyl-3-[5-méthyl-3-[3-(triméthylammonio)propyl]-2(3H)-benzothiazolylidene]-1-propényl]-1-(3-sulfopropyl)-naphtho[1,2-d]thiazolium

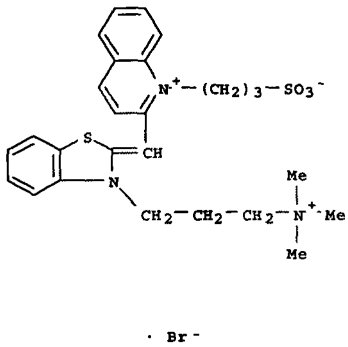

bromure de 1-(3-sulfopropyl)-2-[[3-[3-(trimethylammonio)propyl]-2(3H)-benzothiazolylidene]methyl]-quinolinium)

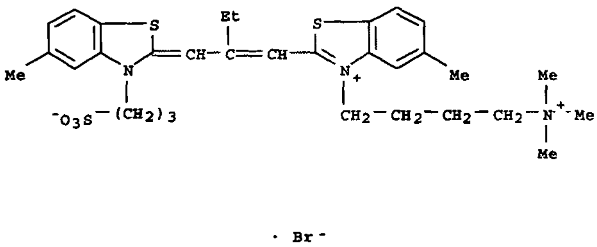

Benzothiazolium, 5-méthyl-2-[2-[[5-méthyl-3-(3-sulfopropyl)-2(3H)-benzothiazolylidene]méthyl]-1-butényl]-3-[4-(triméthylammonio)butyl]-, sel interne, bromure

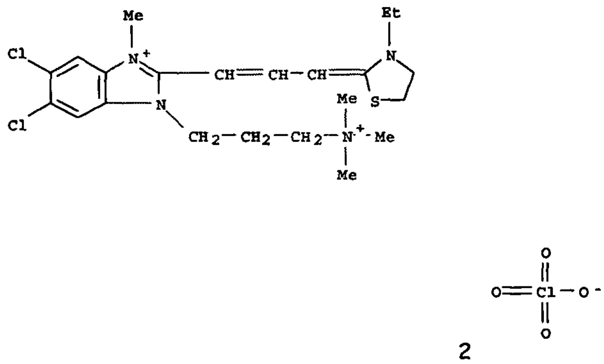

diperchlorate de 5,6-dichloro-2-[3-(3-éthyl-2-thiazolidinylidene)-1-propényl]-1-méthyl-3-[3-(triméthylammonio)propyl]-

1H-benzimidazolium

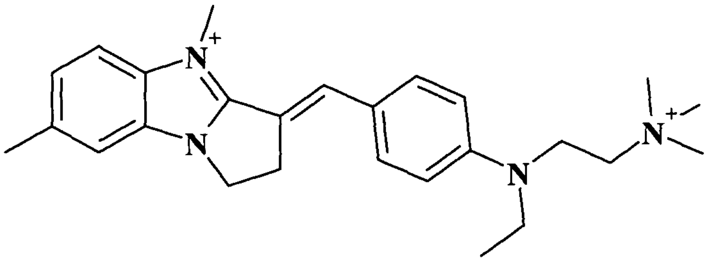

Triméthyl-[2-(méthyl-{2-[4-(4-méthyl-1,2-dihydro-benzo[d]pyrrolo[1,2-a]imidazolium-3ylideneméthyl)-phénylamino]-éthyl}-amino)-ethyl]ammonium

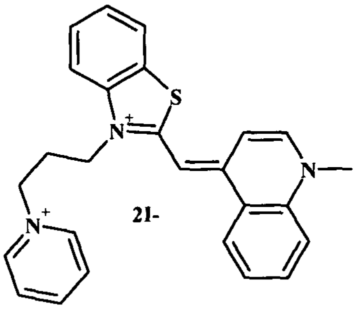

- diiodure de 1-{3-[2-(1-méthyl-1H-quinolin-4-ylideneméthyl)-benzothiazolium-3-yl]-propyl}-pyridinium

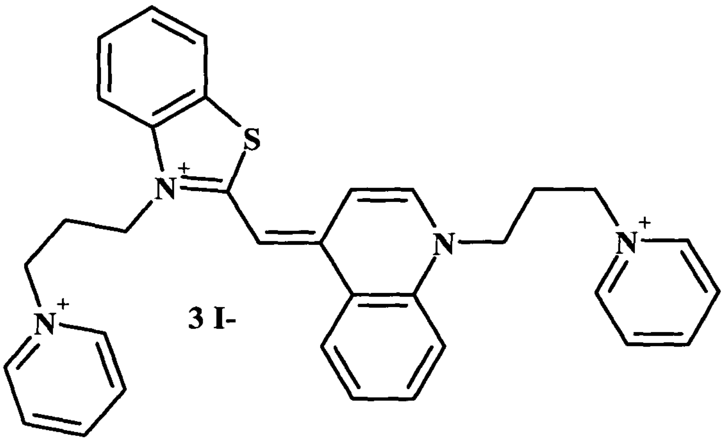

- triiodure de 1-{3-[2-(1-(3-pyridinium-1-yl-propyl)-1H-quinolin-4-ylideneméthyl)-benzothiazolium-3-yl]-propyl}-pyridinium

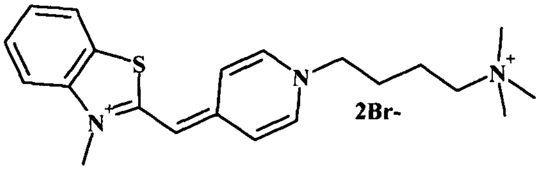

- dibromure de triméthyl-{4-[4-(3-méthyl-benzothiazolium-2-ylméthylène)-4H-pyridin-1-yl]-butyl}-ammonium

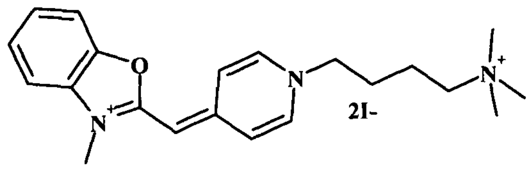

- diiodure de triméthyl-{4-[4-(3-méthyl-benzooxazolium-2-ylméthylène)-4H-pyridin-1-yl]-butyl}-ammonium.

La composition selon la présente demande peut comprendre de 0,001 à 20%, de préférence de 0,05 à 10%, et de manière encore préférée de 0,1 à 5 % en poids de colorant(s) direct(s) de formule (I) par rapport au poids total de la composition.
Les colorants de l'invention peuvent être préparés selon des réactions chimiques connues en soi à partir de chromophores porteurs d'une charge cationique capables de réagir avec le groupement hydrocarboné azoté choisi. Les colorants peuvent notamment être préparés par le procédé décrit dans le document US 3,649,162.
La composition tinctoriale conforme à l'invention peut en outre contenir un ou plusieurs colorants directs additionnels autres que les colorants directs de formule (I) décrits ci-dessus, pouvant être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.
A titre d'exemples de colorants directs additionnels, on peut citer les colorants azoïques cationiques décrits dans les demandes de brevets WO 95/15144, WO-95/01772 et EP-714954.
Parmi ces composés, on peut tout particulièrement citer les colorants suivants :

- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques additionnels les colorants suivants, décrits dans le COLOUR INDEX INTERNATIONAL 3e édition :

- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

Le ou les colorants directs additionnels représentent de préférence de 0,001 à 20% en poids environ du poids total de la composition prête à l'emploi et encore plus préférentiellement de 0,005 à 10% en poids environ.
A titre d'exemple, les bases d'oxydation présentes dans les composition selon l'invention sont choisies parmi les phénylènediamines, les bis-phénylalkylènediamines, les paraaminophénols, les ortho-aminophénols, les bases hétérocycliques autres que les paraphénylènediamines hétérocycliques de formule (I) et leurs sels d'addition.
Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylénediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol;

ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl

1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

La composition de la présente invention peut en outre comprendre un ou plusieurs précurseurs de colorant d'oxydation choisis parmi les coupleurs.

Les coupleurs peuvent être choisis parmi les coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en une quantité allant de 0,001 à 20 % en poids du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

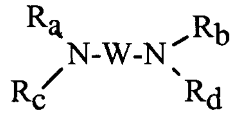

(II)

dans laquelle W est un reste propylène éventuellement substitué par un groupement hydroxyle ou un radical alkyle en $C_1$-$C_4$ ; Ra, Rb, Rc et Rd, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La mise en oeuvre du procédé selon la présente demande comprend l'application d'une composition tinctoriale selon la présente demande sur les fibres kératiniques, puis une étape consistant à laisser poser pendant une période suffisante pour permettre la coloration des cheveux, cette période est généralement comprise entre 5 minutes et 1 heure et de préférence entre 15 minutes et 1 heure.

Le procédé de teinture des fibres kératiniques peut comprendre une étape dans laquelle on utilise un agent oxydant à pH acide, neutre ou alcalin. L'agent oxydant peut être ajouté à la composition de l'invention juste au moment de l'emploi ou il peut être mis en oeuvre à partir d'une composition oxydante le contenant, appliqué simultanément ou séquentiellement à la composition de l'invention.

Ce procédé peut notamment être mis en oeuvre lorsque la composition selon l'invention comprend au moins un précurseur de coloration d'oxydation.

Selon un mode de réalisation particulier, la composition selon la présente invention comprenant au moins un précurseur de colorant d'oxydation est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pause de 5 minutes à 1 heure environ, de préférence 15 minutes à 1 heure environ, les fibres kératiniques sont rincées, lavées au shampooing, rincées à nouveau puis séchées.

Les agents oxydants classiquement utilisés pour la teinture des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Dans le mode de réalisation particulier de l'invention où on mélange la composition tinctoriale avec une composition oxydante, le pH de la composition oxydante renfermant l'agent oxydant est tel qu'après mélange avec la composition tinctoriale, le pH de la composition résultante appliquée sur les fibres kératiniques varie de préférence entre 3 et 12 environ, et de préférence entre 5 et 11 environ et de manière encore plus préférée entre 6 et 8,5. Il peut être ajusté à la valeur désirée au moyen d'agents régulateurs de pH, acidifiants ou alcalinisants habituellement utilisés en teinture des fibres kératiniques et tels que définis précédemment.

La composition appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

La présente demande a également pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent sont destinés à illustrer l'invention, sans toutefois présenter un caractère limitatif.

**Exemple 1**

| Composition tinctoriale pour cheveux | |
|---|---|
| Colorant (1) | 2,5.$10^{-3}$ mole |
| Hydroxyéthylcellulose | 1,44g |
| Parabens | 0,06g |
| Decyl glucoside | 10g |
| Alcool benzylique | 8g |
| PEG 6 | 12g |
| Eau qsp | 100g |
| (1) diiodure de 1- {3-[2-(1-méthyl-1H-quinolin-4-ylideneméthyl)-benzothiazolium-3-yl]-propyl}-pyridinium | |

Après 30 minutes de pause sur des cheveux gris à 90% de blancs et rinçage, on obtient une teinte orangé.

**Exemple 2**

| Composition tinctoriale pour cheveux | |
|---|---|
| Colorant (1) | 2,5.$10^{-3}$ mole |
| Hydroxyéthylcellulose | 1,44g |
| Parabens | 0,06g |
| Decyl glucoside | 10g |
| Alcool benzylique | 8g |
| PEG 6 | 12g |
| Eau qsp | 100g |
| (1) triiodure de 1-{3-[2-(1-(3-pyridinium-1-yl-propyl)-1H-quinolin-4-ylideneméthyl)-benzothiazolium-3-yl]-propyl}-pyridinium | |

Après 30 minutes de pause sur des cheveux gris à 90% de blancs et rinçage, on obtient une teinte rouge orangé.

**Exemple 3**

| Composition tinctoriale pour cheveux | |
|---|---|
| Colorant (1) | 2,5.$10^{-3}$ mole |
| Hydroxyéthylcellulose | 1,44g |
| Parabens | 0,06g |
| Decyl glucoside | 10g |
| Alcool benzylique | 8g |
| PEG 6 | 12g |

(suite)

| | |
|---|---|
| Eau qsp | 100g |
| (1) dibromure de triméthyl-{4-[4-(3-méthyl-benzothiazolium-2-ylméthylène)-4H-pyridin-1-yl]-butyl}-ammonium | |

Après 30 minutes de pause sur des cheveux gris à 90% de blancs et rinçage, on obtient une teinte jaune.

**Exemple 4**

| **Composition tinctoriale pour cheveux** | |
|---|---|
| Colorant (1) | $2{,}5.10^{-3}$ mole |
| Hydroxyéthylcellulose | 1,44g |
| Parabens | 0,06g |
| Decyl glucoside | 10g |
| Alcool benzylique | 8g |
| PEG 6 | 12g |
| Eau qsp | 100g |
| (1) diiodure de triméthyl-{4-[4-(3-méthyl-benzooxazolium-2-ylméthylène)-4H-pyridin-1-yl]-butyl}-ammonium | |

Après 30 minutes de pause sur des cheveux gris à 90% de blancs et rinçage, on obtient une teinte jaune.

**Exemple 5**

| **Composition tinctoriale pour cheveux** | |
|---|---|
| Colorant (1) | 0,15g |
| Ethanol | 10g |
| Alkyl polyglucoside | 5g de matière active |
| 2-amino,2-méthylpropanol | qsp pH 8,0 |
| Eau qsp | 100g |
| (1) acétate de triméthyl-{3-[4-(1-méthyl-naphtho [1,2-d]thiazolium-2-ylméthylène)-4H-pyrimidin-1-yl]-propyl}-ammonium | |

**Exemple 6**

| **Composition tinctoriale pour cheveux** | |
|---|---|
| Colorant (1) | 0,15g |
| Ethanol | 10g |
| Alkyl polyglucoside | 5g de matière active |
| 2-amino,2-méthylpropanol | qsp pH 8,0 |

(suite)

| | |
|---|---|
| Eau qsp | 100g |
| (1) triiodure de 3-[3-(triméthylammonio)propyl]-2-[5-[3-[3-(triméthylammonio)propyl]-2 (3H)-benzothiazolylidene]-1,3-pentadienyl]-benzothiazolium | |

**Exemple 7**

| Composition tinctoriale pour cheveux | |
|---|---|
| Colorant (1) | 0,15g |
| Ethanol | 10g |
| Alkyl polyglucoside | 5g de matière active |
| 2-amino,2-méthylpropanol | qsp pH 8,0 |
| Eau qsp | 100g |
| (1) diiodure de 3-méthyl-2-[5-[1-[3-(triméthylammonio)propyl]-4(1H)-pyridinylidene]-1,3-pentadiényl]-benzoxazolium | |

## Revendications

**1.** Utilisation à titre de colorant direct dans des compositions tinctoriales pour fibres kératiniques, en particulier les fibres kératiniques humaines telles que les cheveux, ou pour la fabrication de telles compositions, d'un composé de formule (I),

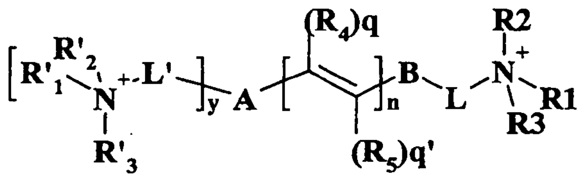

$zX^{z'-}$

(I)

dans laquelle :

- y désigne 0 ou 1 ;
- n désigne un nombre entier allant de 1 à 4 ;
- q et q' désignent indépendamment l'un de l'autre 0 ou 1 ;
- $X^{z'}$- désigne un anion organique ou minéral ;
- z et z' sont tels que la charge globale de la molécule soit nulle ;
- un seul ou les deux atomes de carbone extrêmes du groupement

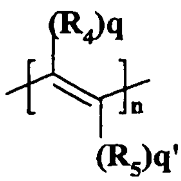

font partie des cycles A et/ou B ;

- A et/ou B désignent indépendamment l'un de l'autre un groupement cyclique non hétérocyclique aromatique ou un groupement hétérocyclique comportant de 5 à 15 chaînons, substitué ou non, l'un au moins des groupements A ou B étant porteur d'au moins une charge positive à la condition que si A est un cycle non-hétérocyclique, alors B désigne un groupement benzothiazolium, naphthothiazolium, naphthoxazolium, benzoxazolium, benzimidazolium, thiazolium, pyrimidobenzimidazolium, benzopyrroloimidazolium, pyrido benzoxazolium, pyrido benzothiazolium, pyrrolo benzoxazolium, pyrrolo benzothiazolium, naphtothiazolo pyridinium, azépino thiazolium, diazepino benzoxazolium, azepino benzoxazolium, pyrido benzoxazolium, azocino benzoxazolium, azocino benzothiazolium, azepino benzothiazolium, ou quinolinium.
- L et L' désignent indépendamment l'un de l'autre une chaîne alkylène linéaire, ramifiée ou cyclique comportant de 1 à 30 atomes de carbone et/ou une chaîne aromatique comportant de 6 à 30 atomes de carbone, substituée ou non ; la chaîne pouvant être ou non interrompue ou terminée par un ou plusieurs groupements hétéroatomiques choisi parmi O, S, NH, NR avec R représentant un radical alkyle en C1-C20, linéaire ou ramifié éventuellement substitué, ou interrompue par un ou plusieurs radicaux cycliques, aromatiques ou non, hétérocycliques ou non, la chaîne elle-même pouvant être porteuse d'une ou plusieurs charges cationiques quaternaires et un ou plusieurs atomes de carbone de la chaîne pouvant être remplacés par un groupement carbonyle.
- R1, R2, R3, R'1, R'2, et R'3 désignent indépendamment l'un de l'autre un radical alkyle en C1-C20 linaire, ramifié ou cyclique éventuellement substitué, deux de ces radicaux ou plus pouvant former avec l'atome d'azote les portant et éventuellement l'un des atomes de carbone ou d'azote de L pour R1, R2 et/ou R3 ou L' pour R'1, R'2 et/ou R'3, un hétérocycle saturé, insaturé ou aromatique, éventuellement substitué pouvant contenir un ou plusieurs hétéroatomes supplémentaires ; un radical aryle en C6-C30 éventuellement substitué.
- R4 et R5 désignent indépendamment l'un de l'autre un atome d'hydrogène, un atome d'halogène, un radical aryle en C6-C30 éventuellement substitué, un radical alkyle en C1-C10 linaire, ramifié ou cyclique éventuellement substitué ; les radicaux R4 et R5 peuvent former avec les atomes de carbone les portant un cycle de 5 à 10 chaînons.

**2.** Composition tinetoriale pour la teinture des fibres kératiniques, en particulier des fibres kératiniques humaines telles que les cheveux comprenant dans un milieu de teinture approprié au moins une base d'oxydation et au moins un composé de formule (I) tel que défini dans la revendication 1.

**3.** Composition selon la revendication 2, **caractérisée en ce qu'**elle comprend de 0,001 à 20%, de préférence de 0,05 à 10%, et de manière encore préférée de 0,1 à 5% en poids de colorant(s) direct(s) de formule (I) par rapport au poids total de la composition.

**4.** Composition tinctoriale selon la revendication 2 ou 3, **caractérisée en ce que** la base d'oxydation est choisie parmi les paraphénylènediamines, les bis-phénylalkylènediamines, les paraaminophénols, les orthoaminophénols, les bases hétérocycliques et leurs sels d'addition.

**5.** Composition tinctoriale selon l'une des revendications 2 à 4, **caractérisée en ce que** la ou les bases d'oxydation sont présentes en une quantité comprise entre 0,001 à 20 % en poids et de préférence entre 0,005 et 6 % en poids par rapport au poids total de la composition.

**6.** Composition selon l'une des revendications 2 à 5, **caractérisée en ce qu'**elle comprend au moins un colorant direct additionnel.

**7.** Composition selon l'une des revendications 2 à 6, **caractérisée en ce qu'**elle contient au moins un précurseur de colorant d'oxydation choisi parmi les coupleurs.

**8.** Composition selon la revendication 7, **caractérisée en ce que** le coupleur est choisi parmi les métaphénylènediamines, les métaaminophénols, les métadiphénols, les coupleurs naphtaléniques, les coupleurs hétérocycliques et leurs sels d'addition.

**9.** Composition selon la revendication 7, **caractérisée en ce que** le coupleur est choisi parmi le 1,3-dihydroxy benzène, le 1,3-dihydroxy 2-méthyl benzène, le 4-chloro -1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-0-hydroxyéthylamino-3,4-méthylènedioxybenzène, 1'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

10. Composition selon l'une des revendications 7 à 9, **caractérisée en ce que** le ou les coupleurs sont présents en une quantité comprise entre 0,001 et 20 % de préférence entre 0,005 et 6 % en poids par rapport au poids total de la composition.

11. Composition selon l'une des revendications 2 à 10, **caractérisée en ce qu'**elle comprend au moins un solvant.

12. Composition selon la revendication 11, **caractérisé en ce que** le solvant est choisi parmi l'éthanol, le propylène glycol, le glycérol, et les monoéthers de polyols.

13. Composition selon l'une des revendications 2 à 12, **caractérisée en ce qu'**elle comprend au moins un adjuvant choisi parmi les tensioactifs anioniques, cationiques, non ioniques, amphotères, zwitterioniques ou leurs mélanges, les polymères anioniques, cationiques, non ioniques, amphotères, zwitterioniques ou leurs mélanges, les agents épaississants minéraux ou organiques, les agents antioxydants, les agents de pénétration, les agents séquestrants, les parfums, les tampons, les agents dispersants, les agents conditionneurs, les agents filmogènes, les céramides, les agents conservateurs, les agents opacifiants.

14. Composition selon l'une des revendications 2 à 13, **caractérisée en ce qu'**elle comprend au moins un agent oxydant choisi parmi le peroxyde d'hydrogène, le peroxyde d'urée, les bromates de métaux alcalins, les persels, les peracides et les enzymes oxydases.

15. Composition selon la revendication 14, **caractérisée en ce que** l'agent oxydant est le peroxyde d'hydrogène.

16. Procédé de teinture des fibres kératiniques, **caractérisé en ce qu'**il comprend l'application d'une composition selon l'une des revendications 2 à 15, sur les fibres kératiniques, puis une étape consistant à laisser poser pendant une période comprise entre 5 minutes et une heure, de préférence entre 15 minutes et 1 heure.

17. Utilisation d'une composition selon l'une des revendications 2 à 15 pour la teintures des fibres kératiniques, en particulier des fibres kératiniques humaines telles que les cheveux.

18. Utilisation d'une composition selon l'une des revendications 2 à 15 sur les des fibres kératiniques, en particulier les fibres kératiniques humaines telles que les cheveux, pour obtenir des teintures présentant une bonne résistance aux agents extérieurs et aux shampoings.

**Claims**

1. Use, as direct dye in compositions for dyeing keratin fibres, in particular human keratin fibres such as the hair, or for the manufacture of such compositions, of a compound of formula (I)

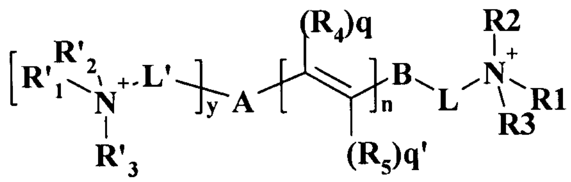

$zX^{z'-}$

(I)

in which:

- y denotes 0 or 1;
- n denotes an integer ranging from 1 to 4;
- q and q' denote, independently of each other, 0 or 1;

- $X^{z'}$ denotes an organic or mineral anion;
- z and z' are such that the overall charge of the molecule is zero;
- only one or both of the extreme carbon atoms of the group

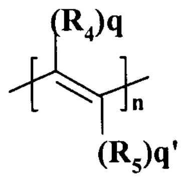

form(s) part of the rings A and/or B;
- A and/or B denotes(s), independently of each other, an aromatic non-heterocyclic cyclic group or a 5- to 15-membered, substituted or unsubstituted heterocyclic group, at least one of the groups A or B bearing at least one positive charge on condition that, if A is a non-heterocyclic ring, then B denotes a benzothiazolium, naphthothiazolium, naphthoxazolium, benzoxazolium, benzimidazolium, thiazolium, pyrimidobenzimidazolium, benzopyrroloimidazolium, pyrido benzoxazolium, pyrido benzothiazolium, pyrrolo benzoxazolium, pyrrolo benzothiazolium, naphthothiazolo pyridinium, azepino thiazolium, diazepino benzoxazolium, azepino benzoxazolium, pyrido benzoxazolium, azocino benzoxazolium, azocino benzothiazolium, azepino benzothiazolium or quinolinium group;
- L and L' denote, independently of each other, a linear, branched or cyclic alkylene chain containing from 1 to 30 carbon atoms and/or a substituted or unsubstituted aromatic chain containing from 6 to 30 carbon atoms; the chain possibly being interrupted or ending with one or more heteroaromatic groups chosen from O, S, NH and NR where R represents an optionally substituted, linear or branched C1-C20 alkyl radical, or interrupted with one or more heterocyclic or non-heterocyclic, aromatic or non-aromatic cyclic radicals, the chain itself possibly bearing one or more quaternary cationic charges and one or more carbon atoms of the chain possibly being replaced with a carbonyl group;
- R1, R2, R3, R'1, R'2 and R'3 denote, independently of each other, a linear, branched or cyclic, optionally substituted C1-C20 alkyl radical, two or more of these radicals possibly forming, with the nitrogen atom bearing them and optionally one of the carbon or nitrogen atoms of L for R1, R2 and/or R3 or L' for R'1, R'2 and/or R'3, an optionally substituted saturated, unsaturated or aromatic heterocycle that may contain one or more additional hetero atoms; an optionally substituted C6-C30 aryl radical;
- R4 and R5 denote, independently of each other, a hydrogen atom, a halogen atom, an optionally substituted C6-C30 aryl radical, or a linear, branched or cyclic, optionally substituted C1-C10 alkyl radical; the radicals R4 and R5 may form with the carbon atoms bearing them a 5- to 10-membered ring.

2. Dye composition for dyeing keratin fibres, in particular human keratin fibres such as the hair, comprising, in a suitable dyeing medium, at least one oxidation base and at least one compound of formula (I) as defined in Claim 1.

3. Composition according to Claim 2, **characterized in that** it comprises from 0.001% to 20%, preferably from 0.05% to 10% and even more preferably from 0.1% to 5% by weight of direct dye(s) of formula (I) relative to the total weight of the composition.

4. Dye composition according to Claim 2 or 3, **characterized in that** the oxidation base is chosen from para-phenylenediamines, bis(phenyl)alkylenediamines, para-aminophenols, ortho-aminophenols and heterocyclic bases, and the addition salts thereof.

5. Dye composition according to any one of Claims 2 to 4, **characterized in that** the oxidation base(s) is (are) present in an amount of between 0.001% and 20% by weight and preferably between 0.005% and 6% by weight relative to the total weight of the composition.

6. Composition according to one of Claims 2 to 5, **characterized in that** it comprises at least one additional direct dye.

7. Composition according to one of Claims 2 to 6, **characterized in that** it contains at least one oxidation dye precursor chosen from couplers.

8. Composition according to Claim 7, **characterized in that** the coupler is chosen from meta-phenylenediamines,

meta-aminophenols, meta-diphenols, naphthalene-based couplers and heterocyclic couplers, and the addition salts thereof.

**9.** Composition according to Claim 7, **characterized in that** the coupler is chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureido-aniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof.

**10.** Composition according to one of Claims 7 to 9, **characterized in that** the coupler(s) is (are) present in an amount of between 0.001% and 20% and preferably between 0.005% and 6% by weight relative to the total weight of the composition.

**11.** Composition according to one of Claims 2 to 10, **characterized in that** it comprises at least one solvent.

**12.** Composition according to Claim 11, **characterized in that** the solvent is chosen from ethanol, propylene glycol, glycerol and polyol monoethers.

**13.** Composition according to one of Claims 2 to 12, **characterized in that** it comprises at least one adjuvant chosen from anionic, cationic, nonionic, amphoteric or zwitterionic surfactants or mixtures thereof, anionic, cationic, nonionic, amphoteric or zwitterionic polymers or mixtures thereof, mineral or organic thickeners, antioxidants, penetrators, sequestering agents, fragrances, buffers, dispersants, conditioning agents, film-forming agents, ceramides, preserving agents and opacifiers.

**14.** Composition according to one of Claims 2 to 13, **characterized in that** it comprises at least one oxidizing agent chosen from hydrogen peroxide, urea peroxide, alkali metal bromates, persalts, peracids and oxidase enzymes.

**15.** Composition according to Claim 14, **characterized in that** the oxidizing agent is hydrogen peroxide.

**16.** Process for dyeing keratin fibres, **characterized in that** it includes the application of a composition according to one of Claims 2 to 15 to the keratin fibres, followed by a step consisting in leaving the composition on the fibres for a period of between 5 minutes and one hour and preferably between 15 minutes and one hour.

**17.** Use of a composition according to one of Claims 2 to 15 for dyeing keratin fibres, in particular human keratin fibres such as the hair.

**18.** Use of a composition according to one of Claims 2 to 15 on keratin fibres, in particular human keratin fibres such as the hair, to obtain dyeing results that show good resistance to external agents and to shampoo.

### Patentansprüche

**1.** Verwendung einer Verbindung der Formel (I) als Direktfarbstoff in Zusammensetzungen zum Färben von Keratinfasern und insbesondere menschlichen Keratinfasern, wie Haaren, oder für die Herstellung solcher Zusammensetzungen:

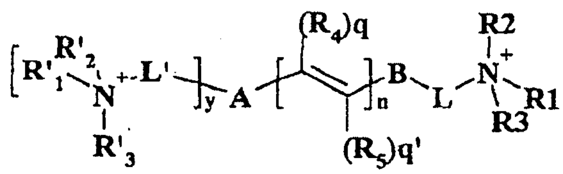

$zX^{z'-}$

(I)

in der Formel:

- y bedeutet 0 oder 1;
- n bedeutet eine ganze Zahl von 1 bis 4;
- q und q' bedeuten unabhängig voneinander 0 oder 1;
- $X^{Z'}$- bedeutet ein organisches oder anorganisches Anion;
- z und z' sind so gewählt, dass die Gesamtladung des Moleküls Null ist;
- nur ein oder beide endständigen Kohlenstoffatome der Gruppe

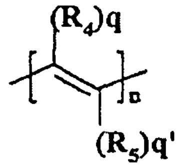

sind Teil der Ringe A und/oder B;
- A und/oder B bedeuten unabhängig voneinander eine nicht heterocyclische, aromatische, cyclische Gruppe oder eine heterocyclische Gruppe mit 5 bis 15 Bestandteilen, die substituiert oder unsubstituiert sind, wobei mindestens eine der Gruppen A oder B mindestens eine positive Ladung trägt, mit der Maßgabe, dass B eine Gruppe Benzothiazolium, Naphthothiazolium, Naphthoxazolium, Benzoxazolium, Benzimidazolium, Thiazolium, Pyrimidobenzimidazolium, Benzopyrroloimidazolium, Pyridobenzoxazolium, Pyridobenzothiazolium, Pyrrolobenzoxazolium, Pyrrolobenzothiazolium, Naphthothiazolopyridinium, Azepinothiazolium, Diazepinobenzoxazolium, Azepinobenzoxazolium, Pyridobenzoxazolium, Azocinobenzoxazolium, Azocinobenzothiazolium, Azepinobenzothiazolium oder Chinolinium bedeutet, wenn A einen nicht heterocyclischen Ring bedeutet;
- L und L' bedeuten unabhängig voneinander eine lineare, verzweigte oder cyclische Alkylenkette mit 1 bis 30 Kohlenstoffatomen und/oder eine aromatische Kette mit 6 bis 30 Kohlenstoffatomen, die gegebenenfalls substituiert ist; wobei die Kette gegebenenfalls durch eine oder mehrere Heteroatomgruppierungen, die unter O, S, NH, NR ausgewählt sind, wobei R eine geradkettige oder verzweigte, gegebenenfalls substituierte $C_{1-20}$-Alkylgruppe bedeutet, unterbrochen oder abgeschlossen werden kann, oder durch eine oder mehrere cyclische, aromatische oder nicht aromatische, heterocyclische oder nicht heterocyclische Gruppen unterbrochen sein kann, wobei die Kette selbst eine oder mehrere quartäre kationische Ladungen tragen kann und ein oder mehrere Kohlenstoffatome der Kette durch eine Carbonylgruppe ersetzt sein können;
- R1, R2, R3, R1, R2 und R3 bedeuten unabhängig voneinander eine lineare, verzweigte oder cyclische Alkylgruppe mit 1 bis 20 Kohlenstoffatomen, die gegebenenfalls substituiert ist; wobei zwei oder mehrere dieser Gruppen mit dem Stickstoffatom, von dem sie getragen werden, und gegebenenfalls einem der Kohlenstoffatome oder Stickstoffatome von L für R1, R2 und/oder R3 oder L' für R'1, R'2 und/oder R'3 einen gesättigten, ungesättigten oder aromatischen, gegebenenfalls substituierten Heterocyclus bilden können, der ein oder mehrere weitere Heteroatome enthalten kann; eine gegebenenfalls substituierte Arylgruppe mit 6 bis 30 Kohlenstoffatomen;
- R4 und R5 bedeuten unabhängig voneinander ein Wasserstoffatom, ein Halogenatom, eine gegebenenfalls substituierte $C_{6-30}$-Arylgruppe, eine lineare, verzweigte oder cyclische $C_{1-10}$-Alkylgruppe, die gegebenenfalls substituiert ist; wobei die Gruppen R4 und R5 mit den Kohlenstoffatomen, von denen sie getragen werden, einen 5- bis 10-gliedrigen Ring bilden können.

2. Farbmittelzusammensetzung zum Färben von Keratinfasern und insbesondere menschlichen Keratinfasern, wie Haaren, die in einem zum Färben geeigneten Medium mindestens eine Oxidationsbase und mindestens eine Verbindung der Formel (I) enthält, wie sie in Anspruch 1 definiert ist.

3. Zusammensetzung nach Anspruch 2, **dadurch gekennzeichnet, dass** sie 0,001 bis 20 %, vorzugsweise 0,05 bis 10 % und noch bevorzugter 0,1 bis 5 Gew.-% Direktfarbstoff(e) der Formel (I), bezogen auf das Gesamtgewicht der Zusammensetzung, enthält.

4. Farbmittelzusammensetzung nach Anspruch 2 oder 3, **dadurch gekennzeichnet, dass** die Oxidationsbase unter den *para*-Phenylendiaminen, Bisphenylalkylendiaminen, para-Aminophenolen, ortho-Aminophenolen, heterocyclischen Basen und deren Additionssalzen ausgewählt ist.

5. Farbmittelzusammensetzung nach einem der Ansprüche 2 bis 4, **dadurch gekennzeichnet, dass** die Oxidationsbase(n) in einer Menge von 0,001 bis 20 Gew.-% und vorzugsweise 0,005 bis 6 Gew.-%, bezogen auf das Gesamtgewicht der Zusammensetzung, enthalten sind.

6. Zusammensetzung nach einem der Ansprüche 2 bis 5, **dadurch gekennzeichnet, dass** sie mindestens einen ergänzenden Direktfarbstoff enthält.

7. Zusammensetzung nach einem der Ansprüche 2 bis 6, **dadurch gekennzeichnet, dass** sie mindestens ein Farbstoffvorprodukt eines Oxidationsfarbstoffes enthält, das unter den Kupplern ausgewählt ist.

8. Zusammensetzung nach Anspruch 7, **dadurch gekennzeichnet, dass** der Kuppler unter den meta-Phenylendiaminen, meta-Aminophenolen, meta-Dihydroxybenzolen, Naphthalinkupplern, heterocyclischen Kupplern und deren Additionssalzen ausgewählt ist.

9. Zusammensetzung nach Anspruch 7, **dadurch gekennzeichnet, dass** der Kuppler unter 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis(2,4-diaminophenoxy)propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxyindol, 4-Hydroxyindol, 4-Hydroxy-N-methylindol, 2-Amino-3-hydroxypyridin, 6-Hydroxybenzomorpholin, 3,5-Diamino-2,6-dimethoxypyridin, 1-N-(β-Hydroxyethyl)amino-3,4-methylendioxybenzol, 2,6-Bis(β-hydroxyethylamino)toluol und ihren Additionssalzen ausgewählt ist.

10. Zusammensetzung nach einem der Ansprüche 7 bis 9, **dadurch gekennzeichnet, dass** der Kuppler oder die Kuppler in einer Menge von 0,001 bis 20 %, vorzugsweise 0,005 bis 6 Gew.-%, bezogen auf das Gesamtgewicht der Zusammensetzung, enthalten sind.

11. Zusammensetzung nach einem der Ansprüche 2 bis 10, **dadurch gekennzeichnet, dass** sie mindestens ein Lösemittel enthält.

12. Zusammensetzung nach Anspruch 11, **dadurch gekennzeichnet, dass** das Lösemittel unter Ethanol, Propylenglycol, Glycerin und Monoethern von Polyolen ausgewählt ist.

13. Zusammensetzung nach einem der Ansprüche 2 bis 12, **dadurch gekennzeichnet, dass** sie mindestens einen Zusatzstoff enthält, der unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, anorganischen oder organischen Verdickungsmitteln, Antioxidantien, Penetrationsmitteln, Maskierungsmitteln, Parfums, Puffern, Dispergiermitteln, Konditioniermitteln, Filmbildnern, Ceramiden, Konservierungsmitteln und Trübungsmitteln ausgewählt ist.

14. Zusammensetzung nach einem der Ansprüche 2 bis 13, **dadurch gekennzeichnet, dass** sie mindestens ein Oxidationsmittel enthält, das unter Wasserstoffperoxid, Harnstoffperoxid, Alkalimetallbromaten, Salzen von Persäuren, Persäuren und Oxidasen ausgewählt ist.

15. Zusammensetzung nach Anspruch 14, **dadurch gekennzeichnet, dass** es sich bei dem Oxidationsmittel um Wasserstoffperoxid handelt.

16. Verfahren zum Färben von Keratinfasern, **dadurch gekennzeichnet, dass** es das Aufbringen einer Zusammensetzung nach einem der Ansprüche 2 bis 15 auf die Keratinfasern und anschließend einen Schritt umfasst, der darin besteht, sie während einer Zeitspanne von 5 Minuten bis 1 Stunde und vorzugsweise 15 Minuten bis 1 Stunde einwirken zu lassen.

17. Verwendung einer Zusammensetzung nach einem der Ansprüche 2 bis 15 zum Färben von Keratinfasern und insbesondere menschlichen Keratinfasern wie Haaren.

18. Verwendung einer Zusammensetzung nach einem der Ansprüche 2 bis 15 an Keratinfasern und insbesondere menschlichen Keratinfasern, wie Haaren, um Färbungen zu bilden, die gegenüber von außen einwirkenden Agentien und Haarwäschen eine hohe Beständigkeit aufweisen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1020176 A2
- EP 1428505 A1
- EP 1415643 A1
- US 3649162 A
- WO 9515144 A
- WO 9501772 A
- EP 714954 A
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2586913